# EUROPEAN PATENT SPECIFICATION

(11) **EP 2 081 933 B1**
(45) Date of publication and mention of the grant of the patent: **23.03.2011**
(21) Application number: 07818515.4
(22) Date of filing: 27.09.2007
(51) Int. Cl.: C07D 487/04

(54) **PYRAZOLOPYRIMIDINES AS PI3K LIPID KINASE INHIBITORS**
PYRAZOLOPYRIMIDINE ALS PI3K-LIPIDKINASEHEMMER
PYRAZOLOPYRIMIDINES UTILISÉES COMME INHIBITEURS DES LIPIDES KINASES Pl3K

(30) Priority: 29.09.2006 EP 06121476
(43) Date of publication of application: 29.07.2009
(73) Proprietor: Novartis AG, 4056 Basel (CH)
(72) Inventor: IMBACH, Patricia, CH-4303 Kaiseraugst (CH); STAUFFER, Frédéric, F-68220 Hesingue (FR); FURET, Pascal, F-68800 Thann (FR); CAPRARO, Hans-Georg, CH-4310 Rheinfelden (CH)
(74) Representative: Dyer, James
(86) International application number: PCT/EP2007/008432
(87) International publication number: WO 2008/037477

(56) References cited:
- WO-A-2007/013673
- WO-A2-2004/052315
- US-A1- 2004 102 452

## Description

The invention relates to novel - at least 3,5-disubstituted - pyrazolo[1,5-a]pyrimidines, processes for the preparation thereof, these compounds for use- alone or in combination with one or more other pharmaceutically active compounds - in the treatment (this term including prophylactic and/or therapeutic treatment) of an inflammatory or obstructive airway disease, such as asthma, disorders commonly occurring in connection with transplantation, or a proliferative disease, such as a tumor disease, which may be solid or liquid, especially one or more of the mentioned diseases which respond to an inhibition of kinases of the PI3-kinase-related protein kinase family, especially lipid kinases and/or PI3 kinase (PI3K) and/or mTOR and/or DNA protein kinase and/or ATM and/or ATR and/or hSMG-1 activity; and the use of such a compound - alone or in combination with one or more other pharmaceutically active compounds - for the manufacture of a pharmaceutical preparation for the treatment of said diseases in animals, especially a human.

US2004/102452 and WO2004/052315 describe pyrazolo[1,5-a]pyrimidines useful for the treatment of inflammatory or proliferative diseases. WO2007/013673 describes nitrogen containing fused heterocyclic compounds as immunosupressive agents.

The - at least 3,5-disubstituted - pyrazolo[1,5-a]pyrimidines preferably are compounds of the formula I,

10.7 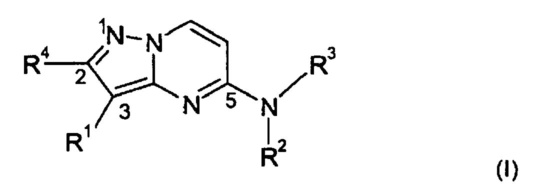 wherein
R¹ is di-C₁-C₇alkoxy-phenyl;
R² is morpholino-C₁-C₇-alkyl, C₁-C₇-alkoxyphenyl, C₁-C₇-alkoxy-halo-phenyl, benzoylaminophenyl, [N'-mono- or N',N'-di-(C₁-C₇-alkyl)-aminocarbonyl]-phenyl, [difluorophenyl-aminocarbonyl]-phenyl, C₁-C₇-alkylcyclohexyl, hydroxyl-C₁-C₇alkyl-cyclohexyl, hydroxyl-cyclohexyl, amino-cyclohexyl, benzyloxycarbonylamino-cyclohexyl, adamantan-1-yl or quinolyl;
R³ is hydrogen or methyl;
or R₂ and R₃ together with the binding nitrogen form piperidino which is unsubstituted or substituted by N-mono- or N,N-di-(C₁-C₇-alkyl)amino, and
R⁴ is hydrogenor a tautomer thereof or an N-oxide thereof, or a pharmaceutically acceptable salt, or a hydrate or solvate thereof.

The general terms used hereinbefore and hereinafter preferably have within the context of this disclosure the following meanings, unless otherwise indicated, where more general terms whereever used may, independently of each other, be replaced by more specific definitions or remain, thus defining more preferred embodiments of the invention:

The prefix "lower" or "C₁-C₇-" denotes a radical having up to and including a maximum of 7, especially up to and including a maximum of 4 carbon atoms, the radicals in question being either linear or branched with single or multiple branching.

Lower alkyl (or C₁-C₇-alkyl) is preferably alkyl with from and including 1 up to and including 7, preferably from and including 1 to and including 4, and is linear or branched; preferably, lower alkyl is butyl, such as n-butyl, sec-butyl, isobutyl, tert-butyl, propyl, such as n-propyl or isopropyl, ethyl or preferably methyl.

The numbering of the positions of substituents at the central pyrazolo[1,5-a]pyrimidines given in the present disclosure (e.g. in the Examples) is provided in formula I above by the small numbers 1, 2, 3 and 5.

Halogen, halogeno (or halo) is especially fluoro, chloro, bromo, or iodo, especially fluoro, chloro or bromo.

Compounds of the formula I may have different isomeric forms. For example, any asymmetric carbon atom may be present in the (R)-, (S)- or (R,S)-configuration, preferably in the (R)- or (S)-configuration. Substituents at a double bond or especially a ring may be present in cis-(= Z-) or trans (= E-) form. The compounds may thus be present as mixtures of isomers or preferably as pure isomers, preferably as pure diastereomers or pure enantiomers.

In unsubstituted or substituted alkyl, alkyl preferably has up to 20, more preferably up to 12, carbon atoms and is especially C₁-C₇-alkyl; is linear or branched one or more times; and is unsubstituted or substituted (in any, e.g. the terminal position) by one or more moieties selected from the substituents mentioned below for aryl, especially from pyrrolidinyl, piperidinyl, morpholinyl, thiomorpholinyl, S-oxo-thiomorpholinyl, S,S-dioxothiomorpholinyl and piperazinyl.

Generally, in the case of R² substituents on phenyl and cyclohexyl can be in the ortho- or preferably the meta- or para-position, generally in position 2 or preferably 3 or 4 relative to the atom binding to the rest of the molecule.

Where asymmetric carbon atoms are present in substituted cyclohexyl, they can be present as mixture of the R- and S-form or especially in pure R- or S-form.

An N-oxide derivative or pharmaceutically acceptable salt of each of the compounds of the formula I is also within the scope of this invention. For example, a nitrogen ring atom of the quinazole core can form an N-oxide in the presence of a suitable oxidizing agent, e.g. a peroxide, such as m-chloro-perbenzoic acid or hydrogen peroxide.

Wherever a compound or compounds of the formula I are mentioned, this is further also intended to include N-oxides of such compounds, as well as tautomers of such compounds or N-oxides, also where not stated explicitly. Tautomerism may, for example, be present of the keto (or oxo)/enol type, the imine/amine (e.g. imine/enamine) type, the lactim/lactame type or the like.

The term "an N-oxide thereof, a tautomer thereof and/or a pharmaceutically acceptable salt thereof" especially means that a compound of the formula I may be present as such or in mixture with its N-oxide, as tautomer or in e.g. equivalency reaction caused) mixture with its tautomer, or as a salt of the compound of the formula I and/or any of these embodiments.

In view of the close relationship between the novel compounds of the formula I in free form and those in the form of their salts, including those salts that can be used as intermediates, for example in the purification or identification of the novel compounds, any reference to the compounds or a compound of the formula I hereinbefore and hereinafter is to be understood as referring also to one or more salts, as appropriate and expedient, as well as to one or more solvates, e.g. hydrates.

Salts are formed, for example, as acid addition salts, preferably with organic or inorganic acids, from compounds of formula I with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, malonic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 4-toluenesulfonic acid, 2-naphthalenesulfonic acid, 1,5-naphthalene-disulfonic acid, 2- or 3-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

Preferred is an embodiment of the invention that relates to a compound of the formula I, wherein
R¹ is di-C₁-C₇-alkoxy-phenyl;
R² is 3-(morpholino)propyl, 4-C₁-C₇-alkoxyphenyl, 4-chloro-3-methoxyphenyl, 4-benzoylamino-phenyl, 4-[N'-mono- or N',N'-di-(C₁-C₇-alkyl)-aminocarbonyl)-phenyl, 4-[2,4-difluorophenyl-aminocarbonyl]-phenyl, 2-, 3- or 4-C₁-C₇-alkylcyclohexyl, 2-, 3- or 4-hydroxyl-C₁-C₇-alkyl-cyclohexyl, 2-, 3- or 4-hydroxyl-cyclohexyl, 2-, 3- or 4-amino-cyclohexyl, 2-, 3- or 4-benzyloxycarbonylamino-cyclohexyl, adamantan-1-yl or quinolyl;
R³ is hydrogen or methyl;
or R₂ and R₃ together with the binding nitrogen form piperidino which is unsubstituted or substituted in the 4-position by N-mono- or N,N-di-(C₁-C₇-alkyl)amino, and R⁴ is hydrogen,
or a tautomer thereof or an N-oxide thereof, or a (preferably pharmaceutically acceptable) salt, or a hydrate or solvate thereof.

Very preferred are also embodiments of the invention represented in the claims.

The invention relates especially to a compound of the formula I as mentioned below in the examples by their names, preferably the isomers shown as formulae, respectively, or a pharmaceutically acceptable salt thereof, or to a compound of the formula I for use according to the invention.

Quite unexpectedly, it has now been found that the compounds of formula I have advantageous pharmacological properties and inhibit the activity of the lipid kinases, such as the PI3-kinase and/or members of the PI3-kinase-related protein kinase family (also called PIKK and include DNA-PK, ATM, ATR, hSMG-1 and mTOR), such as the DNA protein-kinase, and may be used to treat disease or disorders which depend on the activity of said kinases.

The phosphatidylinositol-3'-OH kinase (PI3K) pathway is one of the central signaling pathways that exerts its effect on numerous cellular functions including cell cycle progression, proliferation, motility, metabolism and survival. An activation of receptor tyrosine kinases causes PI3K to phosphorylate phosphatidylinositol-(4,5)-diphosphate, resulting in membrane-bound phosphatidylinositol-(3,4,5)-triphosphate. The latter promotes the transfer of a variety of protein kinases from the cytoplasm to the plasma membrane by binding of phosphatidylinositol-(3,4,5)-triphosphate to the pleckstrin-homology (PH) domain of the kinase. Kinases that are key downstream targets of PI3K include phosphoinositide-dependent kinase 1 (PDK1) and AKT (also known as Protein Kinase B). Phosphorylation of such kinases then allows for the activation or deactivation of numerous other pathways, involving mediators such as GSK3, mTOR, PRAS40, FKHD, NF-κB, BAD, Caspase-9, and the like. An important negative feedback mechanism for the PI3K pathway is PTEN, a phosphatase that catalyses the dephosphorylation of phosphatidylinositol-(3,4,5)-triphosphate to phosphorylate phosphatidylinositol-(4,5)-diphosphate. In more than 60 % of all solid tumors, PTEN is mutated into an inactive form, permitting a constitutive activation of the PI3K pathway. As most cancers are solid tumors, such an observation provides evidence that a targeting of PI3k itself or individual downstream kinases in the PI3K pathway provide a promising approach to mitigate or even abolish the dysregulation in many cancers and thus restore normal cell function and behaviour. This, however, does not exclude that other mechanisms may be responsible for the beneficial effects of PI3K activity modifying agents such as those in the present invention.

Having regard to their inhibitory effect on phosphatidylinositol 3-kinase enzymes, compounds of formula (I) in free or pharmaceutically acceptable salt form, are useful in the treatment of conditions which are mediated by the activation (including normal activity or especially overactivity) of one or more of the members of the PI3 kinase family, especially PI3 kinase enzyme, such as proliferative, inflammatory or allergic conditions, obstructive airways diseases and/or disorders commonly occurring in connection with transplantation.

"Treatment" in accordance with the invention may be therapeutic, e.g. symptomatic, and/or prophylactic. Preferred is the treatment of warm-blooded animals, especially humans.

A compound of formula I is useful in the treatment of a proliferative disease selected from a benign or malignant tumor, carcinoma of the brain, kidney, liver, adrenal gland, bladder, breast, stomach, gastric tumors, ovaries, colon, rectum, prostate, pancreas, lung, vagina or thyroid, sarcoma, glioblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, lymphomas, a mammary carcinoma or a leukemia. Other diseases include Cowden syndrome, Lhermitte-Dudos disease and Bannayan-Zonana syndrome, or diseases in which the PI3K/PKB pathway is aberrantly activated.

Compounds according to the invention are also of use in the treatment of inflammatory or obstructive airways (respiratory tract) diseases, resulting, for example, in reduction of tissue damage, airways inflammation, bronchial hyperreactivity, remodeling or disease progresssion. Inflammatory or obstructive airways diseases to which the present invention is applicable include asthma of whatever type or genesis including both intrinsic (non-allergic) asthma and extrinsic (allergic) asthma, e.g. mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma can be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Compounds of the formula I can be of use for other inflammatory or obstructive airways diseases and conditions to which the present invention is applicable and include acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy.

Compounds of the formula I can be of use for the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to their anti-inflammatory activity, in particular in relation to inhibition of eosinophil activation, compounds of the invention are also of use in the treatment of eosinophil related disorders, e.g. eosinophilia, in particular eosinophil related disorders of the airways

(e.g. involving morbid eosinophilic infiltration of pulmonary tissues) including hypereosinophilia as it effects the airways and/or lungs as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction.

Compounds of the invention are also of use in the treatment of inflammatory or allergic conditions of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphigus, epidermolysis bullosa acquisita, and other inflammatory or allergic conditions of the skin.

Compounds of the invention may also be used for the treatment of other diseases or conditions, such as diseases or conditions having an inflammatory component, for example, treatment of diseases and conditions of the eye such as conjunctivitis, keratoconjunctivitis sicca, and vernal conjunctivitis, diseases affecting the nose including allergic rhinitis, and inflammatory disease in which autoimmune reactions are implicated or having an autoimmune component or aetiology, including autoimmune haematological disorders (e.g. haemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary billiary cirrhosis, uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy).

Furthermore, the invention provides a compound for use according to the definitions herein, or a pharmaceutically acceptable salt, or a hydrate or solvate thereof for the preparation of a medicament for the treatment of a proliferative disease, an inflammatory disease, an obstructive respiratory disease, or a disorder commonly occurring in connection with transplantation.

The invention relates to a compound of the formula I (or a pharmaceutical formulation comprising a compound of the formula I) for use in the treatment of one or more of the diseases mentioned above and below where the disease(s) respond or responds (in a beneficial way, e.g. by partial or complete removal of one or more of its symptoms up to complete cure or remission) to an inhibition of one or more kinases of the PI3-kinase-related protein kinase family, most especially PI3 kinase (PI3K), especially where the kinase shows (in the context of other regulatory mechanisms) inadequately high or more preferably higher than normal (e.g. constitutive) activity.

Whereever the term "use" or "used" is mentioned, this is intended to include a compound of the formula I for use in the prophylactic and/or therapeutic treatment of a disease of a warm-blooded animal, especially a human, preferably of one or more diseases mentioned above or below, the preparation of a pharmaceutical formulation/preparation for use in the prophylactic and therapeutic treatment of a disease mentioned above and below, especially involving mixing a compound of the formula I (as therapeutically active ingredient) with at least one pharmaceutically acceptable carrier material, including making it ready for use in such treatment (e.g. adding an instruction insert (e.g. package leaflet or the like), formulation, appropriate preparation, adaptation for specific uses, customizing and the like), and the compound of the formula I for use in such preparation, and/or for use in all other prophylactic or therapeutic uses mentioned hereinbefore or below. All these aspects are embodiments of the present invention.

The efficacy of the compounds of formula I and salts thereof as PI3 kinase inhibitors can be demonstrated as follows:

The kinase reaction is performed in a final volume of 50 µL per well of a half area COSTAR, 96 well plate. The final concentrations of ATP and phosphatidyl inositol in the assay are 5 µM and 6 µg/mL respectively. The reaction is started by the addition of PI3 kinase p110β.

The components of the assay are added per well as follows:
- 10 µL test compound in 5% DMSO per well in columns 2-1.
- Total activity is determined by addition 10 µL of 5% vol/vol DMSO in the first 4 wells of column 1 and the last 4 wells of column 12.
- The background is determined by addition of 10 µM control compound to the last 4 wells of column 1 and the first 4 wells of column 12.
- 2 mL 'Assay mix' are prepared per plate:
   1.912 mL of HEPES assay buffer
   8.33 µL of 3 mM stock of ATP giving a final concentration of 5 µM per well 1 µL of [³³P]ATP on the activity date giving 0.05 µCi per well 30 µL of 1 mg/mL PI stock giving a final concentration of 6 µg/mL per well 5 µL of 1 M stock MgCl₂ giving a final concentration of 1 mM per well
- 20 µL of the assay mix are added per well.
- 2 mL 'Enzyme mix' are prepared per plate (x* µL PI3 kinase p110β in 2 mL of kinase buffer). The 'Enzyme mix' is kept on ice during addition to the assay plates.
- 20 µl 'Enzyme mix' are added/well to start the reaction.
- The plate is then incubated at room temperature for 90 minutes.
- The reaction is terminated by the addition of 50 µL WGA-SPA bead (wheat germ agglutinin-coated Scintillation Proximity Assay beads) suspension per well.
- The assay plate is sealed using TopSeal-S)heat seal for polystyrene microplates, PerkinElmer LAS (Deutschland) GmbH, Rodgau, Germany) and incubated at room temperature for at least 60 minutes.
- The assay plate is then centrifuged at 1500 rpm for 2 minutes using the Jouan bench top centrifuge (Jouan Inc., Nantes, France).
- The assay plate is counted using a Packard TopCount, each well being counted for 20 seconds.
   * The volume of enzyme is dependent on the enzymatic activity of the batch in use.

Some of the compounds show a certain level of selectivity against the different paralogs PI3K alpha, beta, gamma and delta.

The range of activity in these assays is preferably between 150 nM and about 5 uM.

### Description of biochemical assay for DNA-PK:

The assay is conducted using the kit V7870 from Promega (SignaTECT® DNA-Dependent Protein Kinase Syste, comprises DNA-PK, biotinylated peptide substrate end further ingredients, Promega, Madison, Wisconsin, USA), that quantitates DNA-dependent protein kinase activity, both in purified enzyme preparations and in cell nuclear extracts. DNA-PK is a nuclear serine/threonine protein kinase that requires double-stranded DNA (dsDNA) for activity. The binding of dsDNA to the enzyme results in the formation of the active enzyme and also brings the substrate closer to the enzyme, allowing the phosphorylation reaction to proceed.

DNA-PK X5 reaction buffer (250 mM HEPES, 500 mM KCI, 50 mM MgCl₂, 1 mM EGTA, 0.5 mM EDTA, 5 mM DTT, pH to 7.5 with KOH) is diluted 1/5 in deionised water and BSA (stock = 10 mg/ml) is added to a final concentration of 0.1 mg/ml.

The activation buffer is made from 100 µg/ml of calf thymus DNA in control buffer (10 mM Tris-HCl (pH 7.4), 1 mM EDTA (pH 8.0)). Per tube, the reaction mix is composed of: 2.5 µl of activation or control buffers, 5 µl of X5 reaction buffer, 2.5 µl of p53-derived biotinylated peptide substrate (stock= 4mM), 0.2 µl of BSA (stock at 10 mg/ml) and 5 µl of [γ-³²P] ATP (5 µl of 0.5 mM cold ATP + 0.05 µl of Redivue [γ-³²P] ATP = Amersham AA0068-250 µCi, 3000Ci/mmol, 10 µCi/µl (now GE Gealthcare Biosciences AB, Uppsala, Sweden).

The DNA-PK enzyme (Promega V5811, concentration=100 U/µL) is diluted 1/10 in X1 reaction buffer and kept on ice until imminent use. 10.8 µl of the diluted enzyme is incubated with 1.2 µl of 100 µM compounds (diluted 1/100 in water from 10 mM stock in neat DMSO) for 10 minutes, at room temperature. During that time, 15.2 µl of the reaction mix is added to screw-capped tubes, behind Perspex glass. 9.8 µl of the enzyme is then transferred to the tubes containing the reaction mix and after 5 minutes incubation, at 30°C, the reaction is stopped by adding 12.5 µl of termination buffer (7.5 M guanidine hydrochloride).

After mixing well, a 10 µl aliquot of each tube is spotted onto a SAM2^{®} biotin capture membrane (Promega, Madison, Wisconsin, USA), which is left to dry for a few minutes. The membrane is then washed extensively to remove the excess free [γ-³²P] ATP and nonbiotinylated proteins: once for 30 seconds in 200 ml of 2M NaCl, 3 times for 2 minutes each in 200 ml of 2M NaCl, 4 times for 2 minutes each in 2M NaCl in 1% H₃PO₄ and twice for 30 seconds each in 100 ml of deionised water. The membrane is subsequently left to air-dry at room temperature for 30-60 minutes.

Each membrane square is separated using forceps and scissors and placed into a scintillation vial, after which 8 ml of scintillation liquid (Flo-Scint 6013547 from Perkin-Elmer) is added. The amount of ³²P incorporated into the DNA-PK biotinylated peptide substrate is then determined by liquid scintillation counting. In this test system, compounds of the formula I can be shown to have IC₅₀values in the range from 1 nM to 50 µM, e.g. from 1 nM to 10 µM.

The efficacy of the compounds of the invention in blocking the activation of the PI3K/PKB pathway can be demonstrated in cellular settings as follows:

### Protocol for the detection of phospho-PKB in U87MG cells by Elisa:

U87MG cells (human glioblastoma, ATCC No. HTB-14) are trypsinized, counted in a CASY cell counter (Schärffe systems, Göttingen, Germany), diluted in fresh complete DMEM high glucose medium to load ,per well, 150µL cell suspension containing 4×10⁴ cells, and test plates incubated for 18 hours. In parallel, 50 µL of coating antibody, at the desired concentration in PBS/O is loaded in each well of the ELI SA plates, and plates are kept for 2 h at room temperature. This ELISA assays is performed in black flat-bottom 96-well plates (Microtest^{™}, Falcon Becton-Dickinson, Ref: 353941) sealed with Plate Sealers (Costar-Corning, Ref: 3095). Medium in plates is discarded and replaced by complete DMEM high glucose medium containing either 0.1 % DMSO or 0.1% inhibitor at titers (7) between 10 mM and 0.156 mM in DMSO. After 30 minutes of contact, the medium is quickly removed by aspiration, plates are then placed on ice and immediately cells lyzed with 70 µL of Lysis buffer. In parallel, the 96 wells plates prepared with the coating antibody (1/250 diluted (in PBS/O) Anti-Akt1 C-20, goat, Santa-Cruz-1618, Santa Cruz Biotechnology, Inc., Santa Cruz, California, USA) are washed 3 times 1 min with PBS/O containing 0.05% Tween 20 and 0.1 % Top-Block^{®} (derivative of gelatine that blocks unspecific binding sites on surfaces; Sigma-Aldrich, Fluka, Buchs, Switzerland, Ref.: 37766), and remaining protein binding sites blocked to prevent non-specific interactions with 200 µL of PBS containing 3% Top Block®, for 2 h at room temperature. Well content is replaced with 50 µL of samples from treated cells, and plates are incubated for 3 h at 4°C. The ELISA assays are always done in parallel with the following controls, in 6 replicates: U87MG (untreated control) or Lysis buffer alone (LB). After 3 x 15 minutes washes, all wells received 50 µL of the secondary antibody (1/250 diluted (in 3% top block) Anti-S473P-PKB, rabbit, Cell Signaling-9271, Cell Signaling Technologies, Inc., Danvers, Massachusetts, USA)), and are incubated for 16 h at 4°C. After three washes, plates are incubated with the third and conjugated antibody (1/1000 diluted (in 3% top block) anti rabbit (HRP) Jackson Immuno Research 111-035-144) for 2 hours at room temperature. Finally, the immune-complexes are washed 2 times 15 seconds with PBS/O/ tween20 /top block,1 time with 200µl of water and finally 200µl of water are left in each test well before a for 45 min incubation in darkness. The plates are then assayed with (SuperSignal^{®} ELISA pico Chemiluminescent substrate, Pierce, Ref: 27070, Pierce Biotechnology, Inc., Rockford, Illinois, USA). 100 µL of substrate are added, and plates shacked for 1 min. The luminescence is read immediately on a Top-Count NXT (Packard Bioscience) luminometer. Using this test system, IC₅₀ values in the range from 20 µM to 30 nM, more preferably from 10 µM to 30 nM, can be found for compounds of the formula I as test compounds.

There are also experiments that can demonstrate the antitumor activity of compounds of the formula (I) *in vivo.*

For example, female Harlan (Indianapolis, Indiana, USA) athymic nu/nu mice with s.c. transplanted human glioblastoms U87MG tumors can be used to determine the anti-tumor activity of PI3 kinase inhibitors. On day 0, with the animals under peroral Forene® (1-chloro-2,2,2-trifluoroethyldifluormethylether, Abbot, Wiesbaden, Germany) narcosis, a tumor fragment of approximately 25 mg is placed under the skin on the animals' left flank and the small incised wound is closed by means of suture clips. When tumors reach a volume of 100 mm³, the mice are divided at random into groups of 6-8 animals and treatment commences. The treatment is carried out for a 2-3 weeks period with peroral, intravenous or intraperitoneal administration once daily (or less frequently) of a compound of formula (I) in a suitable vehicle at defined doses. The tumors are measured twice a week with a slide gauge and the volume of the tumors is calculated.

As an alternative to cell line U87MG, other cell lines may also be used in the same manner, for example,
● the MDA-MB 468 breast adenocarcinoma cell line (ATCC No. HTB 132; see also In Vitro 14, 911-15 [1978]);
● the MDA-MB 231 breast carcinoma cell line (ATCC No. HTB-26; see also In Vitro 12, 331 [1976]);
● the MDA-MB 453 breast carcinoma cell line (ATCC No.HTB-131);
● the Colo 205 colon carcinoma cell line (ATCC No. CCL 222; see also Cancer Res. 38, 1345-55 [1978]);
● the DU145 prostate carcinoma cell line DU 145 (ATCC No. HTB 81; see also Cancer Res. 37, 4049-58 [1978]),
● the PC-3 prostate carcinoma cell line PC-3 (especially preferred; ATCC No. CRL 1435; see also Cancer Res. 40, 524-34 [1980]) and the PC-3M prostate carcinoma cell line;
● the A549 human lung adenocarcinoma (ATCC No. CCL 185; see also Int. J. Cancer 17, 62-70 [1976]),
● the NCI-H596 cell line (ATCC No. HTB 178; see also Science 246, 491-4 [1989]);
● the pancreatic cancer cell line SUIT-2 (see Tomioka et al., Cancer Res. 61, 7518-24 [2001]).

Compounds of the invention exhibit T cell inhibiting activity. More particular the compounds of the invention prevent T cell activation and/or proliferation in e.g. aqueous solution, e.g. as demonstrated in accordance with the following test method. The two-way MLR is performed according to standard procedures (J. Immunol. Methods, 1973, 2, 279 and Meo T. et al., Immunological Methods, New York, Academic Press, 1979, 227-39). Briefly, spleen cells from CBA and BALB/c mice (1.6 x 105 cells from each strain per well in flat bottom tissue culture microtiter plates, 3.2 x 105 in total) are incubated in RPMI medium containing 10% FCS, 100 U/ml penicillin, 100 µg/ml streptomycin (Gibco BRL, Basel, Switzerland), 50 µM 2-mercaptoethanol (Fluka, Buchs, Switzerland) and serially diluted compounds. Seven threefold dilution steps in duplicates per test compound are performed. After four days of incubation, 1 µCi 3H-thymidine is added. Cells are harvested after an additional five-hour incubation period, and incorporated 3H-thymidine is determined according to standard procedures. Background values (low control) of the MLR are the proliferation of BALB/c cells alone. Low controls are subtracted from all values. High controls without any sample are taken as 100% proliferation. Percent inhibition by the samples is calculated, and the concentrations required for 50% inhibition (IC50 values) are determined. In this assay, the compounds of the invention preferably have IC50 values in the range of 1 nM to 5 µM, preferably from 5 nM to 500 nM.

A compound of the formula (I) may also be used to advantage in combination with other antiproliferative compounds. Such antiproliferative compounds include, but are not limited to aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibittors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (TEMODAL®); kinesin spindle protein inhibitors, such as SB715992 or SB743921 from GlaxoSmithKline, or pentamidine/chlorpromazine from CombinatoRx; MEK inhibitors such as ARRY142886 from Array PioPharma, AZD6244 from AstraZeneca, PD181461 from Pfizer, leucovorin, EDG binders, antileukemia compounds, ribonucleotide reductase inhibittors, S-adenosylmethionine decarboxylase inhibitors, antiproliferative antibodies or other chemotherapeutic compounds. Further, alternatively or in addition they may be used in combination with other tumor treatment approaches, including surgery, ionizing radiation, photodynamic therapy, implants, e.g. with corticosteroids, hormones, or they may be used as radiosensitizers. Also, in anti-inflammatory and/or antiproliferative treatment, combination with anti-inflammatory drugs is included. Combination is also possible with antihistamine drug substances, bronchodilatatory drugs, NSAID or antagonists of chemokine receptors.

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane can be administered, e.g., in the form as it is marketed, e.g. under the trademark AROMASIN. Formestane can be administered, e.g., in the form as it is marketed, e.g. under the trademark LENTARON. Fadrozole can be administered, e.g., in the form as it is marketed, e.g. under the trademark AFEMA. Anastrozole can be administered, e.g., in the form as it is marketed, e.g. under the trademark ARIMIDEX. Letrozole can be administered, e.g., in the form as it is marketed, e.g. under the trademark FEMARA or FEMAR. Aminoglutethimide can be administered, e.g., in the form as it is marketed, e.g. under the trademark ORIMETEN. A combination of the invention comprising a chemotherapeutic agent which is an aromatase inhibitor is particularly useful for the treatment of hormone receptor positive tumors, e.g. breast tumors.

The term "antiestrogen" as used herein relates to a compound which antagonizes the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX. Raloxifene hydrochloride can be administered, e.g., in the form as it is marketed, e.g. under the trademark EVISTA. Fulvestrant can be formulated as disclosed in US 4,659,516 or it can be administered, e.g., in the form as it is marketed, e.g. under the trademark FASLODEX. A combination of the invention comprising a chemotherapeutic agent which is an antiestrogen is particularly useful for the treatment of estrogen receptor positive tumors, e.g. breast tumors.

The term "anti-androgen" as used herein relates to any substance which is capable of inhibiting the biological effects of androgenic hormones and includes, but is not limited to, bicalutamide (CASODEX), which can be formulated, e.g. as disclosed in US 4,636,505.

The term "gonadorelin agonist" as used herein includes, but is not limited to abarelix, goserelin and goserelin acetate. Goserelin is disclosed in US 4,100,274 and can be administered, e.g., in the form as it is marketed, e.g. under the trademark ZOLADEX. Abarelix can be formulated, e.g. as disclosed in US 5,843,901.

The term "topoisomerase I inhibitor" as used herein includes, but is not limited to topotecan, gimatecan, irinotecan, camptothecian and its analogues, 9-nitrocamptothecin and the macromolecular camptothecin conjugate PNU-166148 (compound A1 in WO99/17804). Irinotecan can be administered, e.g. in the form as it is marketed, e.g. under the trademark CAMPTOSAR. Topotecan can be administered, e.g., in the form as it is marketed, e.g. under the trademark HYCAMTIN.

The term "topoisomerase II inhibitor" as used herein includes, but is not limited to the anthracyclines such as doxorubicin (including liposomal formulation, e.g. CAELYX), daunorubicin, epirubicin, idarubicin and nemorubicin, the anthraquinones mitoxantrone and losoxantrone, and the podophillotoxines etoposide and teniposide. Etoposide can be administered, e.g. in the form as it is marketed, e.g. under the trademark ETOPOPHOS. Teniposide can be administered, e.g. in the form as it is marketed, e.g. under the trademark VM 26-BRISTOL. Doxorubicin can be administered, e.g. in the form as it is marketed, e.g. under the trademark ADRIBLASTIN or ADRIAMYCIN. Epirubicin can be administered, e.g. in the form as it is marketed, e.g. under the trademark FARMORUBICIN. Idarubicin can be administered, e.g. in the form as it is marketed, e.g. under the trademark ZAVEDOS. Mitoxantrone can be administered, e.g. in the form as it is marketed, e.g. under the trademark NOVANTRON.

The term "microtubule active compound" relates to microtubule stabilizing, microtubule destabilizing compounds and microtublin polymerization inhibitors including, but not limited to taxanes, e.g. paclitaxel and docetaxel, vinca alkaloids, e.g., vinblastine, especially vinblastine sulfate, vincristine especially vincristine sulfate, and vinorelbine, discodermolides, cochicine and epothilones and derivatives thereof, e.g. epothilone B or D or derivatives thereof. Paclitaxel may be administered e.g. in the form as it is marketed, e.g. TAXOL. Docetaxel can be administered, e.g., in the form as it is marketed, e.g. under the trademark TAXOTERE. Vinblastine sulfate can be administered, e.g., in the form as it is marketed, e.g. under the trademark VINBLASTIN R.P.. Vincristine sulfate can be administered, e.g., in the form as it is marketed, e.g. under the trademark FARMISTIN. Discodermolide can be obtained, e.g., as disclosed in US 5,010,099. Also included are Epothilone derivatives which are disclosed in WO 98110121, US 6,194,181, WO 98/25929, WO 98/08849, WO 99/43653, WO 98/22461 and WO 00/31247. Especially preferred are Epothilone A and/or B.

The term "alkylating compound" as used herein includes, but is not limited to, cyclophosphamide, ifosfamide, melphalan or nitrosourea (BCNU or Gliadel). Cyclophosphamide can be administered, e.g., in the form as it is marketed, e.g. under the trademark CYCLOSTIN. Ifosfamide can be administered, e.g., in the form as it is marketed, e.g. under the trademark HOLOXAN.

The term "histone deacetylase inhibitors" or "HDAC inhibitors" relates to compounds which inhibit the histone deacetylase and which possess antiproliferative activity. This includes compounds disclosed in WO 02/22577, especially N-hydroxy-3-[4-[[(2-hydroxyethyl)[2-(1H-indol-3-yl)ethyl]-amino]methyl]phenyl]-2E-2-propenamide, N-hydroxy-3-[4-[[[2-(2-methyl-1*H*-indol-3-yl)-ethyl]-amino]methyl]phenyl]-2*E*-2-propenamide and pharmaceutically acceptable salts thereof. It further especially includes Suberoylanilide hydroxamic acid (SAHA).

The term "antineoplastic antimetabolite" includes, but is not limited to, 5-Fluorouracil or 5-FU, capecitabine, gemcitabine, DNA demethylating compounds, such as 5-azacytidine and decitabine, methotrexate and edatrexate, and folic acid antagonists such as pemetrexed. Capecitabine can be administered, e.g., in the form as it is marketed, e.g. under the trademark XELODA. Gemcitabine can be administered, e.g., in the form as it is marketed, e.g. under the trademark GEMZAR.

The term "platin compound" as used herein includes, but is not limited to, carboplatin, cis-platin, cisplatinum and oxaliplatin. Carboplatin can be administered, e.g., in the form as it is marketed, e.g. under the trademark CARBOPLAT. Oxaliplatin can be administered, e.g., in the form as it is marketed, e.g. under the trademark ELOXATIN.

The term "compounds targeting/decreasing a protein or lipid kinase activity"; or a "protein or lipid phosphatase activity"; or "further anti-angiogenic compounds" as used herein includes, but is not limited to, protein tyrosine kinase and/or serine and/or threonine kinase inhibitors or lipid kinase inhibitors, e.g.,
a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g. a N-phenyl-2-pyrimidine-amine derivative, e.g. imatinib, SU101, SU6668 and GFB-111;
b) compounds targeting, decreasing or inhibiting the activity of the fibroblast growth factor-receptors (FGFR);
c) compounds targeting, decreasing or inhibiting the activity of the insulin-like growth factor receptor I (IGF-IR), such as compounds which target, decrease or inhibit the activity of IGF-IR, especially compounds which inhibit the kinase activity of IGF-I receptor, such as those compounds disclosed in WO 02/092599, or antibodies that target the extracellular domain of IGF-I receptor or its growth factors;
d) compounds targeting, decreasing or inhibiting the activity of the Trk receptor tyrosine kinase family, or ephrin B4 inhibitors;
e) compounds targeting, decreasing or inhibiting the activity of the Axl receptor tyrosine kinase family;
f) compounds targeting, decreasing or inhibiting the activity of the Ret receptor tyrosine kinase;
g) compounds targeting, decreasing or inhibiting the activity of the Kit/SCFR receptor tyrosine kinase, e.g. imatinib;
h) compounds targeting, decreasing or inhibiting the activity of the C-kit receptor tyrosine kinases - (part of the PDGFR family), such as compounds which target, decrease or inhibit the activity of the c-Kit receptor tyrosine kinase family, especially compounds which inhibit the c-Kit receptor, e.g. imatinib;
i) compounds targeting, decreasing or inhibiting the activity of members of the c-Abl family, their gene-fusion products (e.g. BCR-Abl kinase) and mutants, such as compounds which target decrease or inhibit the activity of c-Abl family members and their gene fusion products, e.g. a N-phenyl-2-pyrimidine-amine derivative, e.g. imatinib or nilotinib (AMN107); PD180970; AG957; NSC 680410; PD173955 from ParkeDavis; or dasatinib (BMS-354825)
j) compounds targeting, decreasing or inhibiting the activity of members of the protein kinase C (PKC) and Raf family of serine/threonine kinases, members of the MEK, SRC, JAK, FAK, PDK1, PKB/Akt, and Ras/MAPK family members, and/or members of the cyclin-dependent kinase family (CDK) and are especially those staurosporine derivatives disclosed in US 5,093,330, e.g. midostaurin; examples of further compounds include e.g. UCN-01, safingol, BAY 43-9006, Bryostatin 1, Perifosine; Ilmofosine; RO 318220 and RO 320432; GO 6976; Isis 3521; LY333531/LY379196; isochinoline compounds such as those disclosed in WO 00/09495; FTIs; PD184352 or QAN697 (a P13K inhibitor) or AT7519 (CDK inhibitor);
k) compounds targeting, decreasing or inhibiting the activity of protein-tyrosine kinase inhibitors, such as compounds which target, decrease or inhibit the activity of protein-tyrosine kinase inhibitors include imatinib mesylate (GLEEVEC) or tyrphostin. A tyrphostin is preferably a low molecular weight (Mr < 1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5-dihydroxyphenyl)methyl]amino}-benzoic acid adamantyl ester; NSC 680410, adaphostin);
l) compounds targeting, decreasing or inhibiting the activity of the epidermal growth factor family of receptor tyrosine kinases (EGFR, ErbB2, ErbB3, ErbB4 as homo- or heterodimers) and their mutants, such as compounds which target, decrease or inhibit the activity of the epidermal growth factor receptor family are especially compounds, proteins or antibodies which inhibit members of the EGF receptor tyrosine kinase family, e.g. EGF receptor, ErbB2, ErbB3 and ErbB4 or bind to EGF or EGF related ligands, and are in particular those compounds, proteins or monoclonal antibodies generically and specifically disclosed in WO 97/02266, e.g. the compound of ex. 39, or in EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/30347 (e.g. compound known as CP 358774), WO 96/33980 (e.g. compound ZD 1839) and WO 95/03283 (e.g. compound ZM105180); e.g. trastuzumab (Herceptin™), cetuximab (Erbitux™), Iressa, Tarceva, OSI-774, Cl-1033, EKB-569, GW-2016, E1.1, E2.4, E2.5, E6.2, E6.4, E2.11, E6.3 or E7.6.3, and 7H-pyrrolo-[2,3-d]pyrimidine derivatives which are disclosed in WO 03/013541; and
m) compounds targeting, decreasing or inhibiting the activity of the c-Met receptor, such as compounds which target, decrease or inhibit the activity of c-Met, especially compounds which inhibit the kinase activity of c-Met receptor, or antibodies that target the extracellular domain of c-Met or bind to HGF.

Further anti-angiogenic compounds include compounds having another mechanism for their activity, e.g. unrelated to protein or lipid kinase inhibition e.g. thalidomide (THALOMID) and TNP-470.

Compounds which target, decrease or inhibit the activity of a protein or lipid phosphatase are e.g. inhibitors of phosphatase 1, phosphatase 2A, or CDC25, e.g. okadaic acid or a derivative thereof.

Compounds which induce cell differentiation processes are e.g. retinoic acid, α-γ- or δ-tocopherol or α-γ- or δ-tocotrienol.

The term cyclooxygenase inhibitor as used herein includes, but is not limited to, e.g. Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g. 5-methyl-2-(2'-chloro-6'-fluoroanilino)phenyl acetic acid, lumiracoxib.

The term "bisphosphonates" as used herein includes, but is not limited to, etridonic, clodronic, tiludronic, pamidronic, alendronic, ibandronic, risedronic and zoledronic acid. "Etridonic acid" can be administered, e.g., in the form as it is marketed, e.g. under the trademark DIDRONEL. "Clodronic acid" can be administered, e.g., in the form as it is marketed, e.g. under the trademark BONEFOS. "Tiludronic acid" can be administered, e.g., in the form as it is marketed, e.g. under the trademark SKELID. "Pamidronic acid" can be administered, e.g. in the form as it is marketed, e.g. under the trademark AREDIA™. "Alendronic acid" can be administered, e.g., in the form as it is marketed, e.g. under the trademark FOSAMAX. "Ibandronic acid" can be administered, e.g., in the form as it is marketed, e.g. under the trademark BONDRANAT. "Risedronic acid" can be administered, e.g., in the form as it is marketed, e.g. under the trademark ACTONEL. "Zoledronic acid" can be administered, e.g. in the form as it is marketed, e.g. under the trademark ZOMETA. The term "mTOR inhibitors" relates to compounds which inhibit the mammalian target of rapamycin (mTOR) and which possess antiproliferative activity such as sirolimus (Rapamune®), everolimus (Certican™), CCI-779 and ABT578.

The term "heparanase inhibitor" as used herein refers to compounds which target, decrease or inhibit heparin sulfate degradation. The term includes, but is not limited to, PI-88.

The term " biological response modifier" as used herein refers to a lymphokine or interferons, e.g. interferon γ.

The term "inhibitor of Ras oncogenic isoforms", e.g. H-Ras, K-Ras, or N-Ras, as used herein refers to compounds which target, decrease or inhibit the oncogenic activity of Ras e.g. a "farnesyl transferase inhibitor" e.g. L-744832, DK8G557 or R115777 (Zarnestra).

The term "telomerase inhibitor" as used herein refers to compounds which target, decrease or inhibit the activity of telomerase. Compounds which target, decrease or inhibit the activity of telomerase are especially compounds which inhibit the telomerase receptor, e.g. telomestatin.

The term "methionine aminopeptidase inhibitor" as used herein refers to compounds which target, decrease or inhibit the activity of methionine aminopeptidase. Compounds which target, decrease or inhibit the activity of methionine aminopeptidase are e.g. bengamide or a derivative thereof.

The term "proteasome inhibitor" as used herein refers to compounds which target, decrease or inhibit the activity of the proteasome. Compounds which target, decrease or inhibit the activity of the proteasome include e.g. Bortezomid (Velcade™) and MLN 341.

The term "matrix metalloproteinase inhibitor" or ("MMP" inhibitor) as used herein includes, but is not limited to, collagen peptidomimetic and nonpeptidomimetic inhibitors, tetracycline derivatives, e.g. hydroxamate peptidomimetic inhibitor batimastat and its orally bioavailable analogue marimastat (BB-2516), prinomastat (AG3340), metastat (NSC 683551) BMS-279251, BAY 12-9566, TAA211, MM1270B or AAJ996.

The term "compounds used in the treatment of hematologic malignancies" as used herein includes, but is not limited to, FMS-like tyrosine kinase inhibitors e.g. compounds targeting, decreasing or inhibiting the activity of FMS-like tyrosine kinase receptors (Flt-3R); interferon, 1-b-D-arabinofuransylcytosine (ara-c) and bisulfan; and ALK inhibitors e.g. compounds which target, decrease or inhibit anaplastic lymphoma kinase.

Compounds which target, decrease or inhibit the activity of FMS-like tyrosine kinase receptors (Flt-3R) are especially compounds, proteins or antibodies which inhibit members of the Flt-3R receptor kinase family, e.g. PKC412, midostaurin, a staurosporine derivative, SU11248 and MLN518.

The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90 e.g., 17-allylamino,17-demethoxygeldanamycin (17AAG), a geldanamycin derivative; other geldanamycin related compounds; radicicol and HDAC inhibitors.

The term "antiproliferative antibodies" as used herein includes, but is not limited to, trastuzumab (Herceptin™), Trastuzumab-DM1,erbitux, bevacizumab (Avastin™), rituximab (Rituxan^{®}), PRO64553 (anti-CD40) and 2C4 Antibody. By antibodies is meant e.g. intact monoclonal antibodies, polyclonal antibodies, multispecific antibodies formed from at least 2 intact antibodies, and antibodies fragments so long as they exhibit the desired biological activity.

For the treatment of acute myeloid leukemia (AML), compounds of formula (I) can be used in combination with standard leukemia therapies, especially in combination with therapies used for the treatment of AML. In particular, compounds of formula (I) can be administered in combination with, e.g., famesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.

The term "antileukemic compounds" includes, for example, Ara-C, a pyrimidine analog, which is the 2'-alpha-hydroxy ribose (arabinoside) derivative of deoxycytidine. Also included is the purine analog of hypoxanthine, 6-mercaptopurine (6-MP) and fludarabine phosphate. Compounds which target, decrease or inhibit activity of histone deacetylase (HDAC) inhibitors such as sodium butyrate and suberoylanilide hydroxamic acid (SAHA) inhibit the activity of the enzymes known as histone deacetylases. Specific HDAC inhibitors include MS275, SAHA, FK228 (formerly FR901228), Trichostatin A and compounds disclosed in US 6,552,065, in particular, *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof and *N*-hydroxy-3-[4-[(2-hydroxyethyl){2-(1*H*-indol-3-yl)ethyl]-amino]methyl]phenyl]-2*E*-2-propenamide, or a pharmaceutically acceptable salt thereof, especially the lactate salt. Somatostatin receptor antagonists as used herein refers to compounds which target, treat or inhibit the somatostatin receptor such as octreotide, and SOM230 (pasireotide).

Tumor cell damaging approaches refer to approaches such as ionizing radiation. The term "ionizing radiation" referred to above and hereinafter means ionizing radiation that occurs as either electromagnetic rays (such as X-rays and gamma rays) or particles (such as alpha and beta particles). Ionizing radiation is provided in, but not limited to, radiation therapy and is known in the art. See Hellman, Principles of Radiation Therapy, Cancer, in Principles and Practice of Oncology, Devita et al., Eds., 4th Edition, Vol. 1, pp. 248-275 (1993).

The term "EDG binders" as used herein refers a class of immunosuppressants that modulates lymphocyte recirculation, such as FTY720.

The term "ribonucleotide reductase inhibitors" refers to pyrimidine or purine nucleoside analogs including, but not limited to, fludarabine and/or cytosine arabinoside (ara-C), 6-thioguanine, 5-fluorouracil, cladribine, 6-mercaptopurine (especially in combination with ara-C against ALL) and/or pentostatin. Ribonucleotide reductase inhibitors are especially hydroxyurea or 2-hydroxy-1*H*-isoindole-1,3-dione derivatives, such as PL-1, PL-2, PL-3, PL-4, PL-5, PL-6, PL-7 or PL-8 mentioned in Nandy et al., Acta Oncologica, Vol. 33, No. 8, pp. 953-961 (1994).

The term "*S*-adenosylmethionine decarboxylase inhibitors" as used herein includes, but is not limited to the compounds disclosed in US 5,461,076.

Also included are in particular those compounds, proteins or monoclonal antibodies of VEGF disclosed in WO 98/35958, e.g. 1-(4-chloroanilino)-4-(4-pyridylmethyl)phthalazine or a pharmaceutically acceptable salt thereof, e.g. the succinate, or in WO 00/09495, WO 00/27820, WO 00/59509, WO 98/11223, WO 00/27819 and EP 0 769 947; those as described by Prewett et al, Cancer Res, Vol. 59, pp. 5209-5218 (1999); Yuan et al., Proc Natl Acad Sci U S A, Vol. 93, pp. 14765-14770 (1996); Zhu et al., Cancer Res, Vol. 58, pp. 3209-3214 (1998); and Mordenti et al., Toxicol Pathol, Vol. 27, No. 1, pp. 14-21 (1999); in WO 00/37502 and WO 94/10202; ANGIOSTATIN, described by O'Reilly et al., Cell, Vol. 79, pp. 315-328 (1994); ENDOSTATIN, described by O'Reilly et al., Cell, Vol. 88, pp. 277-285 (1997); anthranilic acid amides; ZD4190; ZD6474; SU5416; SU6668; bevacizumab; or anti-VEGF antibodies or anti-VEGF receptor antibodies, e.g. rhuMAb and RHUFab, VEGF aptamer e.g. Macugon; FLT-4 inhibitors, FLT-3 inhibitors, VEGFR-2 IgG1 antibody, Angiozyme (RPI 4610) and Bevacizumab (Avastin™).

Photodynamic therapy as used herein refers to therapy which uses certain chemicals known as photosensitizing compounds to treat or prevent cancers. Examples of photodynamic therapy includes treatment with compounds, such as e.g. VISUDYNE and porfimer sodium. Angiostatic steroids as used herein refers to compounds which block or inhibit angiogenesis, such as, e.g., anecortave, triamcinolone, hydrocortisone, 11-α-epihydrocotisol, cortexolone, 17α-hydroxyprogesterone, corticosterone, desoxycorticosterone, testosterone, estrone and dexamethasone.

Implants containing corticosteroids refers to compounds, such as e.g. fluocinolone, dexamethasone.

"Other chemotherapeutic compounds" include, but are not limited to, plant alkaloids, hormonal compounds and antagonists; biological response modifiers, preferably lymphokines or interferons; antisense oligonucleotides or oligonucleotide derivatives; shRNA or siRNA; or miscellaneous compounds or compounds with other or unknown mechanism of action.

The compounds of the invention are also useful as co-therapeutic compounds for use in combination with other drug substances such as anti-inflammatory, bronchodilatory or antihistamine drug substances, particularly in the treatment of obstructive or inflammatory airways diseases such as those mentioned hereinbefore, for example as potentiators of therapeutic activity of such drugs or as a means of reducing required dosaging or potential side effects of such drugs. A compound of the invention may be mixed with the other drug substance in a fixed pharmaceutical composition or it may be administered separately, before, simultaneously with or after the other drug substance. Accordingly the invention includes a combination of a compound of the invention as hereinbefore described with an anti-inflammatory, bronchodilatory, antihistamine or anti-tussive drug substance, said compound of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/035668, WO 03/048181, WO 03/062259, WO 03/064445, WO 03/072592, non-steroidal glucocorticoid receptor agonists such as those described in WO 00/00531, WO 02/10143, WO 03/082280, WO 03/082787, WO 03/104195, WO 04/005229; LTB4 antagonists such LY293111, CGS025019C, CP-195543, SC-53228, BIIL 284, ONO 4057, SB 209247 and those described in US 5451700; LTD4 antagonists such as montelukast and zafirlukast; PDE4 inhibitors such cilomilast (Ariflo® GIaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vemalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204. WO 03/104205, WO 03/39544, WO 04/000814, WO 04/000839, WO 04/ 005258, WO 04/018450, WO 04/018451, WO 04/018457, WO 04/018465, WO 04/018431, WO 04/018449, WO 04/018450, WO 04/018451, WO 04/018457, WO 04/018465, WO 04/ 019944, WO 04/019945, WO 04/045607 and WO 04/037805; A2a agonists such as those disclosed in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/ 039762, WO 04/039766, WO 04/045618 and WO 04/046083; A2b antagonists such as those described in WO 02/42298; and beta-2 adrenoceptor agonists such as albuterol (salbutamol), metaproterenol, terbutaline, salmeterol fenoterol, procaterol, and especially, formoterol and pharmaceutically acceptable salts thereof, and compounds (in free or salt or solvate form) of formula I of WO 0075114, which document is incorporated herein by reference, preferably compounds of the Examples thereof, especially a compound of formula 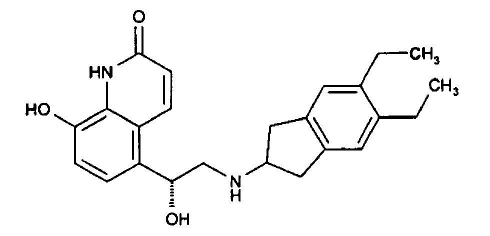 and pharmaceutically acceptable salts thereof, as well as compounds (in free or salt or solvate form) of formula I of WO 04/16601, and also compounds of WO 04/033412. Suitable bronchodilatory drugs include anticholinergic or antimuscarinic compounds, in particular ipratropium bromide, oxitropium bromide, tiotropium salts and CHF 4226 (Chiesi), and glycopyrrolate, but also those described in WO 01/04118, WO 02/51841, WO 02/53564, WO 03/00840, WO 03/87094, WO 04/05285, WO 02/00652, WO 03/53966, EP 424021, US 5171744, US 3714357, WO 03/33495 and WO 04/018422.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in WO 03/099807, WO 04/026841 and JP 2004107299.

Other useful combinations of compounds of the invention with anti-inflammatory drugs are those with antagonists of chemokine receptors, e.g. CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzo-cyclohepten-8-yl[carbonyl]amino]phenyl]-methyl]tetrahydro-N,N-dimethyl-2H-pyran-4-amin-ium chloride (TAK-770), and CCR-5 antagonists described in US 6166037 (particularly claims 18 and 19), WO 00/66558 (particularly claim 8), WO 00/66559 (particularly claim 9), WO 04/018425 and WO 04/026873.

The structure of the active compounds identified by code nos., generic or trade names may be taken from the actual edition of the standard compendium "The Merck Index" or from databases, e.g. Patents International (e.g. IMS World Publications).

The above-mentioned compounds, which can be used in combination with a compound of the formula (I), can be prepared and administered as described in the art, such as in the documents cited above.

By "combination", there is meant either a fixed combination in one dosage unit form, or a kit of parts for the combined administration where a compound of the formula (I) and a combination partner may be administered independently at the same time or separately within time intervals that especially allow that the combination partners show a cooperative, e.g. synergistic effect.

The invention also provides a pharmaceutical preparation, comprising a compound of formu-Ia I as defined herein, or an N-oxide or a tautomer thereof, or a pharmaceutically acceptable salt of such a compound, or a hydrate or solvate thereof, and at least one pharmaceutically acceptable carrier.

A compound of formula I can be administered alone or in combination with one or more other therapeutic compounds, possible combination therapy taking the form of fixed combinations or the administration of a compound of the invention and one or more other therapeutic (including prophylactic) compounds being staggered or given independently of one another, or the combined administration of fixed combinations and one or more other therapeutic compounds. A compound of formula I can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The dose of a compound of the formula I or a pharmaceutical acceptable salt thereof to be administered to warm-blooded animals, for example humans of approximately 70 kg body weight, is preferably from approximately 3 mg to approximately 5 g, more preferably from approximately 10 mg to approximately 1.5 g per person per day, divided preferably into 1 to 3 single doses which may, for example, be of the same size. Usually, children receive half of the adult dose.

The compounds of the invention may be administered by any conventional route, in particular parenterally, for example in the form of injectable solutions or suspensions, enterally, e.g. orally, for example in the form of tablets or capsules, topically, e.g. in the form of lotions, gels, ointments or creams, or in a nasal or a suppository form. Topical administration is e.g. to the skin. A further form of topical administration is to the eye. Pharmaceutical compositions comprising a compound of the invention in association with at least one pharmaceutical acceptable carrier or diluent may be manufactured in conventional manner by mixing with a pharmaceutically acceptable carrier or diluent.

The invention relates also to pharmaceutical compositions comprising an effective amount, especially an amount effective in the treatment of one of the above-mentioned disorders, of a compound of formula I or an N-oxide or a tautomer thereof together with one or more pharmaceutically acceptable carriers that are suitable for topical, enteral, for example oral or rectal, or parenteral administration and that may be inorganic or organic, solid or liquid. There can be used for oral administration especially tablets or gelatin capsules that comprise the active ingredient together with diluents, for example lactose, dextrose, mannitol, and/or glycerol, and/or lubricants and/or polyethylene glycol. Tablets may also comprise binders, for example magnesium aluminum silicate, starches, such as com, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilisers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectionning, dissolving or lyophilising processes, and comprise approximately from 1% to 99%, especially from approximately 1% to approximately 20%, active ingredient(s).

Additionally, the present invention provides a compound of formula I or an N-oxide or a tautomer thereof, or a pharmaceutically acceptable salt of such a compound, for use in the treatment of a disease mentioned herein, most especially in a patient requiring such treatment.

The present invention also relates to the use of a compound of formula I or a tautomer thereof, or a pharmaceutically acceptable salt of such a compound, for the preparation of a medicament for the treatment of a proliferative disease, an inflammatory disease, or an obstructtive airway disease, or disorders commonly occurring in connection with transplantation.

Furthermore, the invention describes a method for the treatment of a proliferative disease which responds to an inhibition of lipid kinases and/or PI3-kinase-related protein kinases, in particular the PI3 kinase, and/or mTOR, and/or DNA protein kinase activity, which comprises administering a compound of formule I or a pharmaceutically acceptable salt thereof, wherein the radicals and symbols have the meanings as defined above, especially in a quantity effective against said disease, to a warm-blooded animal requiring such treatment.

Furthermore, the invention relates to a pharmaceutical composition for treatment of solid or liquid tumours, comprising an antitumor effective dose of a compound of the formula I as described above or a pharmaceutically acceptable salt of such a compound together with a pharmaceutical carrier.

### Manufacturing Process:

The invention relates also to a process for the manufacture of a compound of the formula I, an N-oxide thereof, a tautomer thereof and/or a salt thereof.

Compounds of the formula I can be prepared according to or in analogy to methods that, in principle and with other educts, intermediates and final products, are known in the art, especially and according to the invention by a process comprising
reacting a leaving group carrying compound of the formula II, 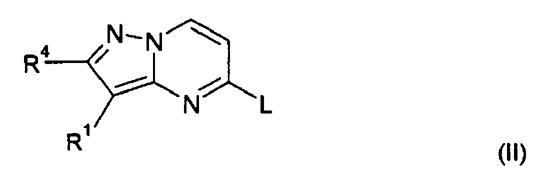 wherein R¹ and R⁴ are as defined for a compound of the formula I and L is a leaving group, with an amino compound of the formula III, 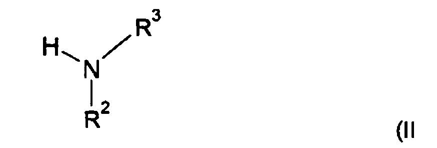 wherein R² and R³ are as defined for a compound of the formula I,
where in the reaction functional groups in the starting materials can be present in protected form and in the obtainable compounds of the formula I carrying one or more protectting groups such protecting groups are removed;
and, if desired, a compound of the formula I obtainable according to the reaction given above is converted into a different compound of the formula I, an obtainable salt of a compound of the formula I is converted into a different salt thereof, an obtainable free compound of the formula I is converted into a salt thereof, and/or an obtainable isomer of a compound of the formula I is separated from one or more different obtainable isomers of the formula I.

### Preferred reaction conditions:

In the following more detailed description of the process, optional reactions and conversions, synthesis of starting materials and intermediates and the like, R¹, R², R³ and R⁴ have the meanings given for a compound of the formula I or the compound mentioned specifically, while L is as defined for a compound of the formula II, respectively.

Where useful or required, the reactions can take place under an inert gas, such as nitrogen or argon.

L is preferably aryl(e.g. toluene)-sulfonyloxy, C₁-C₇-alkane(e.g. methane)-sulfonyloxy or more preferably halo, especially chloro or bromo.

The reaction conditions are preferably chosen from customary conditions of a nucleophilic aromatic substitution, e.g. carrying out the reaction, if required in a sealed vessel (e.g. a seal reaction), in a polar solvent, such as an alcohol, e.g. ethanol, and/or an aprotic solvent, such as 1-methyl-2-pyrrolidone, preferably at a temperature in the range from 120 to 180 °C; preferably, the energy for heating is provided by microwave excitation.

### Protecting groups

If one or more other functional groups, for example carboxy, hydroxy, amino, or mercapto, are or need to be protected in a starting material, e.g. in any one or more starting materials of the formula II or III or other starting materials, intermediates and educts mentioned below,, because they should not take part in the reaction or disturb the reaction, these are such groups as are usually used in the synthesis of peptide compounds, and also of cephalosporins and penicillins, as well as nucleic acid derivatives and sugars. Protecting groups are such groups that are no longer present in the final compounds once they are removed, while groups that remain as substitutents are not protecting groups in the sense used here which is groups that are added at a certain intermediate stage and removed to obtain a final compound. For example, tert-butoxy if remaining in a compound of the formula I is a substituent, while if it is removed to obtain the final compound of the formula I it is a protecting group.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by acetolysis, protonolysis, solvolysis, reduction, photolysis or also by enzyme activity, for example under conditions analogous to physiological conditions, and that they are not present in the end-products. The specialist knows, or can easily establish, which protecting groups are suitable with the reactions mentioned above and below.

The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/l, Georg Thieme Verlag, Stuttgart 1974, in H.-D. Jakubke and H. Jescheit, "Aminosäuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.

### Optional Reactions and Conversions

A compound of the formula I may be converted into a different compounds of the formula I according to standard reaction procedures.

For example, in a compound of the formula I carrying an esterified carboxy group, such as C₁-C₇-alkoxycarbonyl, this esterified carboxy group may be hydrolysed to give the corresponding free carboxy group, e.g. in the presence of a base, such as an alkali metal hydroxide, e.g. lithium hydroxide, in an appropriate solvent, e.g. a cyclic ether, such as dioxane, water or a mixture thereof, e.g. at temperatures in the range from 0 to 50 °C.

In a compound of the formula I carrying a free carboxy group as substituent (e.g. obtainable by a preceding step as described in the last paragraph), this free carboxy group may be converted into a corresponding carbamoyl or N-mono or N,N-di-substituted carbamoyl group, e.g. by reaction with ammonia, N-mono- or N,N-di-(C₁-C₇-alkyl and/or phenyl-C₁-C₇alkyl)-amine, piperidine, piperazine, 4-C₁-C₇-alkyl-piperazine, morpholine, thiomorpholine, S-oxo-thiomorpholine or S,S-dioxothiomorpholine or other educts for groups mentioned above; the reaction preferably takes place with the carboxy group in active form, more preferably under customary condensation conditions, where among the possible reactive derivatives of a carboxy group reactive esters (such as the hydroxybenzotriazole (HOBT), pentafluorophenyl, 4-nitrophenyl or N-hydroxysuccinimide ester), acid halogenides (such as the acid chloride or bromide) or reactive anhydrides (such as mixed anhydrides with lower alkanoic acids or symmetric anhydrides) are preferred. Reactive carbonic acid derivatives can preferably be formed in situ. The reaction is carried out by dissolving the corresponding compounds of the formula I carrying one or more carboxy substituents in a suitable solvent, for example a halogenated hydrocarbon, such as methylene chloride, *N,N*-dimethylformamide, *N,N*-dimethylacetamide, *N*-methyl-2-pyrrolidone, 4-(N,N-dimethylamino)-pyridine or acetonitrile, or a mixture of two or more such solvents, and by the addition of a suitable base, for example triethylamine, diisopropylethylamine (DIPEA) or *N*-methylmorpholine and, if the reactive derivative of the carboxyl substituent(s) is formed *in situ*, a suitable coupling agent that forms a preferred reactive derivative of the carboxy group *in situ*, for example dicyclohexylcarbodiimide/1-hydroxybenzotriazole (DCC/ HOBT); bis(2-oxo-3-oxazolidinyl)phosphinic chloride (BOPCI); O-(1,2-dihydro-2-oxo-1-pyridyl)-*N*,*N*,*N*',*N*'-tetramethyluronium tetrafluoroborate (TPTU); O-benzotriazol-1-yl)-N,N,N',N'-tetramethyluronium tetrafluoroborate (TBTU); (benzotriazol-1-yloxy)-tripyrrolidinophosphonium-hexafluorophosphate (PyBOP), O-(1H-6-chlorobenzotriazole-1-yl)-1,1,3,3-tetramethyluronium hexafluorophosphate, 1-(3-dimethylaminopropyl)-3-ethylcarbodiimide hydrochloride/hydroxybenzotriazole or/1-hydroxy-7-azabenzotriazole (EDC/HOBT or EDC/HOAt) or HOAt alone, or with (1-chloro-2-methylpropenyl)-dimethylamine. For review of some other possible coupling agents, see e.g. Klauser; Bodansky, Synthesis (1972), 453-463. The reaction mixture is preferably stirred at a temperature of between approximately -20 and 50 °C, especially between 0 °C and 30 °C, e.g. at room temperature.

A nitrogen ring atom of the pyrazolo[1,5-a]pyrimidine core or a nitrogen-containing heterocyclic (e.g. heteroaryl) substituent can form an N-oxide in the presence of a suitable oxidizing agent, e.g. a peroxide, such as m-chloro-perbenzoic acid or hydrogen peroxide.

Also in the optional process steps, carried out "if desired", functional groups of the starting compounds which should not take part in the reaction may be present in unprotected form or may be protected for example by one or more of the protecting groups mentioned hereinabove under "protecting groups". The protecting groups are then wholly or partly removed according to one of the methods described there.

Salts of a compound of formula I with a salt-forming group may be prepared in a manner known *per se.* Acid addition salts of compounds of formula I may thus be obtained by treatment with an acid or with a suitable anion exchange reagent.

Salts can usually be converted to free compounds, e.g. by treating with suitable basic compounds, for example with alkali metal carbonates, alkali metal hydrogencarbonates, or alkali metal hydroxides, typically potassium carbonate or sodium hydroxide.

Stereoisomeric mixtures, e.g. mixtures of diastereomers, can be separated into their corresponding isomers in a manner known *per se* by means of suitable separation methods. Diastereomeric mixtures for example may be separated into their individual diastereomers by means of fractionated crystallization, chromatography, solvent distribution, and similar procedures. This separation may take place either at the level of a starting compound or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

It should be emphasized that reactions analogous to the conversions mentioned in this chapter may also take place at the level of appropriate intermediates (and are thus useful in the preparation of corresponding starting materials).

### Starting materials:

The starting materials of the formulae II and III, as well as other starting materials, intermediates or educts mentioned herein, e.g. below, can be prepared according to or in analogy to methods that are known in the art, the materials are known in the art and/or are commercially available, or by or in analogy to methods mentioned in the Examples. Novel starting materials, as well as processes for the preparation thereof, are likewise an embodiment of the present invention. In the preferred embodiments, such starting materials are used and the reaction chosen are selected so as to enable the preferred compounds to be obtained.

Starting materials of the formula III are known in the art, commercially available or can be prepared according to or in analogy to methods known in the art. For example, a compound of the formula III wherein R³ is hydrogen can be obtained from a corresponding compound wherein instead of the amino group a nitro group is present, e.g. with iron powder in ethanol, water and acetic acid at elevated temperatures, e.g. from 30 to 100 °C, or with hydrogen in the presence of a catalyst, e.g. Raney-Ni in methanol at temperatures e.g. from 0 to 50 °C. In both cases other customary solvents are possible. A corresponding compound of the formula III wherein R³ is C₁-C₄-alkyl can then be obtained by alkylation, e.g. with a corresponding C₁-C₄-alkylhalogenide.

A compound of the formula II can, for example, be obtained by reacting an oxo compound of the formula IV, 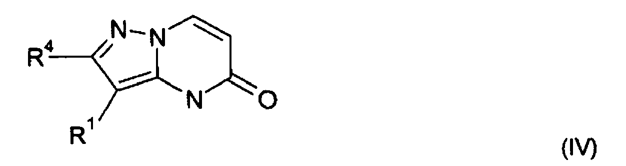 with an inorganic acid halide, such as phosphoroxychloride (POCl₃), e.g. in the absence of a solvent, at elevated temperatures, e.g. in the range from 30 to 140 °C, for example at about 120 °C.

A compound of the formula IV can, for example, be obtained from a pyrazoline compound of the formula V, 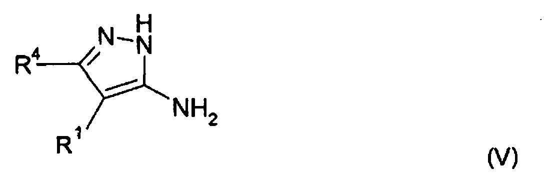 by reaction with propiolic acid ester (e.g. propiolic acid methyl ester) in the presence of an appropriate solvent or solvent mixture (e.g. a cyclic ether, such as dioxane) at elevated temperatures, e.g. in the range from 30 to 130 °C, e.g. at 110 °C.

A compound of the formula V wherein R⁴ is hydrogen, or further methyl or trifluoromethyl, can, for example, be prepared by reaction of a nitrile compound of the formula VI, 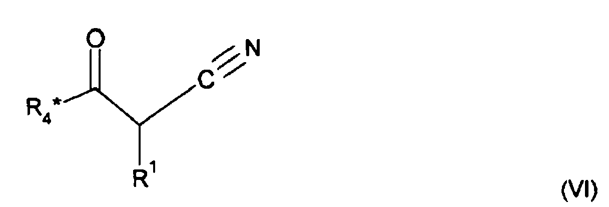 wherein R⁴* is hydrogen or methyl with hydrazine (e.g. as monohydrate) in a carboxylic acid, e.g. acetic acid, and if required an additional solvent, e.g. toluene, preferably at temperatures in the range from 0 to 110 °C.

A compound of the formula VI can, for example, be obtained from a compound of the formula VII, 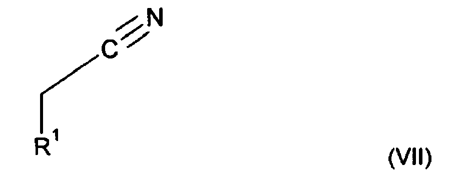 by reaction with ethyl formate (R⁴* = H) or ethyl acetate (R⁴ = methyl) in an appropriate solvent, such as absolute ethanol and/or toluene, at elevated temperatures, e.g. in the range from 30 °C to the reflux temperature of the reaction mixture, in the presence of an alkali metal alcoholate, such as sodium methylate or sodium ethylate.

Examples for the synthesis of compounds of the formula V can be derived from WO 2005/070431.

### Examples:

The following examples illustrate the invention without limiting the scope thereof.

Temperatures are measured in degrees Celsius. Unless otherwise indicated, the reactions take place at RT.

The R_{f} values in TLC indicate the ratio of the distance moved by each substance to the distance moved by the eluent front. R_{f} values for TLC are measured on 5 x 10 cm TLC plates, silica gel F₂₅₄, Merck, Darmstadt, Germany.

### Analytical HPLC conditions:

System 1: Linear gradient 2-100% CH₃CN (0.1%TFA) and H₂O (0.1% TFA) in 7min + 2min 100% CH₃CN (0.1%TFA); detection at 215 nm, flow rate 1 mL/min at 30°C. Column: Nucleosil 100-3 C18HD (125 x 4mm)

Abbreviations:
- d: day(s)
- EtOAc: ethyl acetate
- h: hour(s)
- HPLC: High Performance Liquid Chromatography
- mL: milliliter(s)
- min: minute(s)
- MS(ESI⁺): electrospray mass spectrometry
- NMP: 1-methyl-2-pyrrolidinone
- R_{f}: ratio of fronts in TLC
- RT: room temperature
- TBME: tert. Butyl methyl ether
- TFA: trifluoro acetic acid
- TLC: thin layer chromatography
- tRet: retention time
- UV: Ultraviolet

Starting Materials:
Starting materials are prepared as described or obtained from commercial suppliers; in the latter case, the suppliers mentioned have the following addresses or complete names:
   ABCR = ABCR GmbH & Co., KG, Karlsruhe, Germany;
   ACROS = ACROS Organics BVBA, Geel, Belgium;
   Albany Molecular Research, Inc. (corresponding to AMRI Fine Chemicals) Albany, New York, USA;
   Aldrich = Sigma-Aldrich, Munich, Germany;
   Apin Chemicals. Ltd., Abingston, Oxon, United Kingdom;
   ASTATECH, Inc., Bristol, PA, USA;
   Fluka = Fluka and Riedel-de Haën, Buchs, Switzerland;
   Fluorochem Ltd., Derbyshire, UK.

### Illustrative procedure for the synthesis of educts, shown with the formula and educts for 4-(3,4-dimethon-phenyl)-2H-pyrazol-3-ylamine; details see WO2005070431):

The synthesis of 4-(3,4-Dimethoxy-phenyl)-2H-pyrazol-3-ylamine can be achieved as described in the prior art (see WO20051070431). The following scheme provides as short summary:

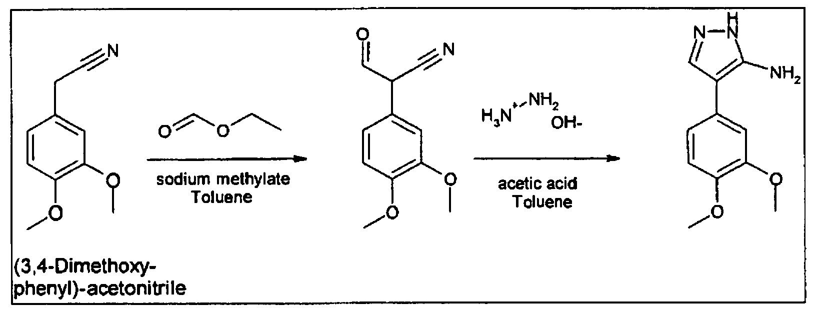

Exemplary synthesis scheme of further intermediates and final compounds for the synthesis of the title compound of Example 1, (1S,2R)-2-[3-(3,4-Dimethoxy-phenyl)-pyrazolo[9,5-a]pyrimidin-5-ylamino]-cyclohexanol (with other substituents, other compounds of the formula I are available analogously):

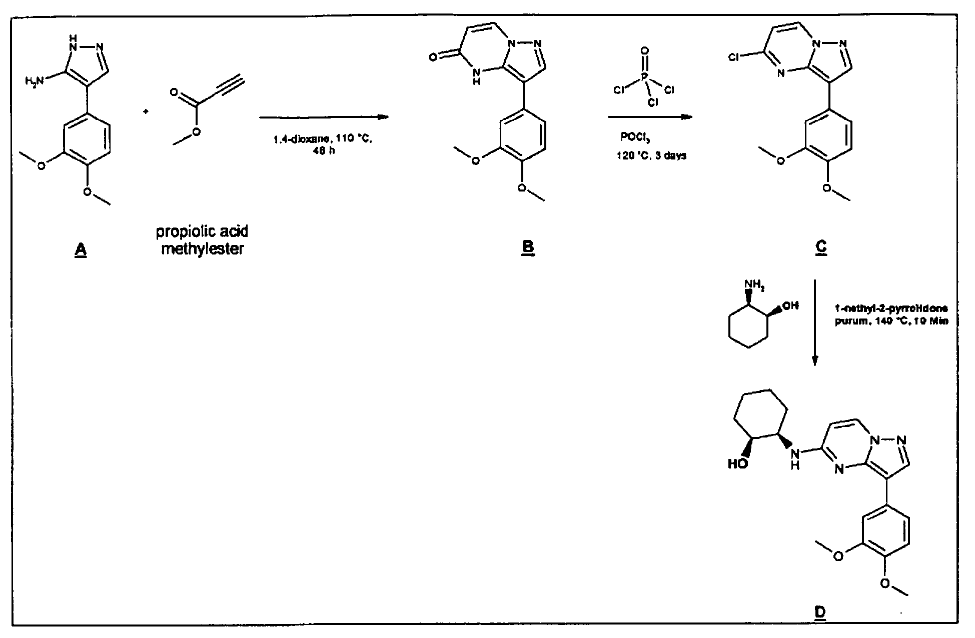

### Example 1: (1S,2R)-2-[3-(3,4-Dimethoxy-phenyl)-pyrazolo[1,5-a]pyrimidin-5-ylamino]-cyclohexanol:

5-Chloro-3-(3,4-dimethoxy-phenyl)-pyrazolo[1,5-a]pyrimidine (C) (25 mg; 0.086 mmol) and (as amino starting material) cis-2-aminocyclohexanol HCl (ACROS, F26585) (125 mg; 1.09 mmol) are suspended in NMP (0.5 mL) and stirred at 140°C for 10 min at 300 W in an Emry Optimizer (microwave oven from Personal Chemistry AB, Uppsala, Sweden). The reaction mixture is treated with water (2 mL) and extracted with EtOAc (2 x 10 mL). The combined organic layers are washed with a saturated NaHCO₃ solution, water, then brine, and then dried (Na₂SO₄), and concentrated under reduced pressure. Purification is done by chromatography on a 4 g Redisep® Sg-100 column (Teledyne ISCO, Inc., Lincoln, Nebraska, USA) eluting with CH₂Cl₂/CH₃OH 95:5 (v/v)), to obtain the title compound as beige crystals: MS(ESI⁺)m/z= 369.1 (M+H)⁺; HPLC: tRet = 5.33 minutes (System 1).

***Stage 1.1:*** 3-(3,4-Dimethoxy-phenyl)-4H-pyrazolo[1,5-a]pyrimidin-5-one (B): 4-(3,4-Dimethoxy-phenyl)-2H-pyrazol-3-ylamine (see WO2005070431) (10g; 45.6 mMol) is suspended in 1,4-dioxane and treated at RT with propiolic acid methylester (4.10 mL); 45.6 mMol). The reaction mixture is stirred at 110°C (bath) for 46 h. After cooling to RT the precipitated product is filtered off, washed with 1,4-dioxane and dried to obtain the title compound as a white solid. Title compound: MS(ESI⁺):m/z= 272.0 (M+H)⁺; HPLC: tRet = 4.43 minutes (System 1).

***Stage 1.2:*** 5-Chloro-3-(3,4-dimethoxy-phenyl)-pyrazolo[1,5-a]pyrimidine: 3-(3,4-Dimethoxy-phenyl)-4H-pyrazolo[1,5-a]pyrimidin-5-one (B) (Example 1; stage 1.1) (1.0 g; 3.69 mmol) is suspended in POCl₃ (17.2 mL; 184 mmol) and stirred for 2 d at 120°C. After cooling to RT the solvent is removed under reduced pressure, the residue is taken up into a saturated solution of NaHCO₃ (70 mL) and extracted with EtOAc (2x200 mL). The combined organic layers are washed with a saturated solution of NaHCO₃, water, brine, dried (Na₂SO₄), and concentrated under reduced pressure. After stirring in diethyl ether, and filtereing off, the title compound is obtained as brown crystals. Title compound: MS(ESI⁺):m/z= 290.0 (M+H)⁺; HPLC: tRet = 5.53 minutes (System 1).

### Example 2: trans-{4-[3-(3,4-Dimethoxy-phenyl)-pyrazolo[1,5-a]pyrimidin-5-ylamino]-cyclohexyl}-carbamic acid benzyl ester:

The title compound is prepared as described in Example 1, but using N-benzyloxycarbonyl-*trans*-1,4-cyclohexane-diamine (ASTATECH INC., 58107) as amino starting material Title compound: white crystals; MS(ESI⁺):m/z= 502.0 (M+H)⁺; HPLC: tRet = 6.98 minutes (System 1).

### Example 3: trans-4-[3-(3,4-Dimethoxy-phenyl)-pyrazolo[1,5-a]pyrimidin-5-ylamino]-cyclohexanol:

The title compound is prepared as described in Example 1, using *trans-*4-aminocyclohexanol (ACROS, 346680250; CAS Nr. 27489-62-9)_as amino starting material Title compound: beige crystals; MS(ESI⁺):m/z= 369.1 (M+H)⁺; HPLC: tRet = 5.01 minutes (System1).

### Example 4: [3-(3,4-Dimethoxy-phenyl)-pyrazolo[1,5-a]pyrimidin-5-yl]-(4-methoxy-phenyl)-amine:

The title compound is prepared as described in Example 1, using p-anisidine (Fluka 10490) as amino starting material Title compound: yellowish crystals; MS(ESI⁺):m/z= 377.0 (M+H)⁺; HPLC: tRet = 6.62 minutes (System1).

### Example 5: trans-N-[3-(3,4-Dimethoxy-phenyl)-pyrazolo[1,5-a]pyrimidin-5-yl]-cyclohexane-1,4-diamine:

The title compound is prepared as described in Example 1, using *trans-*1,4-diaminocyclohexane (Aldrich 33,998-9) as amino starting material Title compound: yellowish crystals; MS(ESI⁺):m/z= 368.1 (M+H)⁺; HPLC: tRet = 4.68 minutes (System 1).

### Example 6: Adamantan-1-yl-[3-(3,4-dimethoxy-phenyl)-pyrazolo[1,5-a]pyrimidin-5-yl]-amine:

The title compound is prepared as described in example 1, using 1-adamantylamine (Aldrich 13,857-6) as amino starting material. Title compound: bright yellow crystals; MS(ESI⁺):m/z= 405 (M+H)⁺; HPLC: tRet = 7.85 minutes (System1).

### Example 7: trans-4-{[3-3,4-Dimethoxy-phenyl)-pyrazolo[1,5-a]pyrimidin-5-yl]-methylamino}-cyclohexanol:

The title compound is prepared as described in Example 1, using *trans-*4-methylamino-cyclohexanol as amino starting material. Title compound: slightly brownish crystals; MS(ESI⁺):m/z= 383.1 (M+H)⁺; HPLC: tRet = 5.79 minutes (System1).

### Stage 7.1: trans- 4-Methylamino-cyclohexanol

tert-Butyl *trans-*4-hydroxycyclohexylcarbamate (AMRI Fine Chemicals A00070) (215 mg; 1.00 mmol) is dissolved in THF anhydrous (10 mL). Under an atmosphere of argon, LiAlH₄ (157 mg; 4.01 mmol) is added portion-wise, slowly at 0°C. After removal of the ice bath, the reaction mixture is let come to RT, followed by heating to 70°C for 15 h. The reaction mixture is cooled to 0°C followed by dropwise addition of 0.22 mL of THF/water (1:1). After this, the mixture is treated dropwise with NaOH (0.4 mL of 4N solution; 1.6 mmol) and water (0.65 mL). The white mixture is filtered, washed with THF (25 mL) and freed from the solvent under reduced pressure to obtain the title compound as white crystals. Title compound: MS(ESI⁺):m/z= 130.0 (M+H)⁺.

### Example 8: [3-(3,4-Dimethoxy-phenyl)-pyrazolo[1,5-a]pyrimidin-5-yl]-(3-morpholin-4-yl-propyl)-amine:

The title compound is prepared as described in Example 1, using using 3-morpholino-propylamine (Aldrich 204-590-2) as amino starting material. Title compound: Slightly orange crystals; MS(ESI⁺):m/z= 398.1 (M+H)⁺; HPLC: tRet = 4.72 minutes (System1).

### Example 9: {1-[3-(3,4-Dimethoxy-phenyl)-pyrazolo[1,5-a]pyrimidin-5-yl]-piperidin-4-yl}-diethyl-amine:

The title compound is prepared as described in Example 1, using 4-diethylamino-piperidine (Fluorochem 021287) as amino starting material. Title compound: slightly brownish crystals; MS(ESI⁺):m/z= 410.1 (M+H)⁺; HPLC: tRet = 4.97 minutes (System1).

### Example 10: cis-N-[3-(3,4-Dimethoxy-phenyl)-pyrazolo[1,5-a]pyrimidin-5-yl]-cyclo-hexane-1,2-diamine:

The title compound is prepared as described in Example 1, using cis-1,2-diamino-cyclohexan (Fluka purum 32845) as amino starting material. Title compound (enantiomeric mixture): slightly brownish solid; MS(ESI⁺):m/z= 368.1 (M+H)⁺; HPLC: tRet = 5.02 minutes (System1).

### Example 11: 4-[3-(3,4-Dimethoxy-phenyl)-pyrazolo[1,5-a]pydmidin-5-ylaminol-N-phenyl-benzamide:

The title compound is prepared as described in Example 1, using 4-aminobenzanilide (Apin Chemicals Ltd, 228a) as amino starting material. Title compound: yellow crystals; MS(ESI⁺):m/z= 466 (M+H)⁺; HPLC: tRet = 6.81 minutes (System1).

### Example 12: N-(2-Diethylamino-ethyl)-4-[3-(3,4-dimethoxy-phenyl)-pyrazolo[1,5-a]pyrimidin-5-ylamino]-benzamide:

The title compound is prepared as described in Example 1, using procainamide HCl (Fluka 81664) as amino starting material. Title compound: yellow crystals; MS(ESI⁺):m/z= 489.0 (M+H)⁺; HPLC: tRet = 5.25 minutes (System1).

### Example 13: trans-{4-[3-(3,4-Dimethoxy-phenyl)-pyrazolo[1,5-a]pyrimidin-5-ylamino]-cyclohexan-1-yl}-methanol:

The title compound is prepared as described in Example 1, using trans-4-hydroxymethyl-cyclohexylamine as amino starting material (prepared as described in Schneider, Wolde-mar; Lehmann, Klaus., Tetrahedron Letters (1970), (49), 4285-8.).. Title compound: white crystals; MS(ESI⁺):m/z= 383.1 (M+H)⁺; HPLC: tRet = 5.71 minutes (System1).

**Example 14:** [3-(3,4-Dimethoxy-phenyl)-pyrazolo[1,5-a]pyrimidin-5-yl]-quinolin-5-yl-amine:

The title compound is prepared as described in Example 1, using 5-aminoquinoline (Fluka 07340) as amino starting material. Title compound: beige crystals; MS(ESI⁺):m/z= 398.0 (M+H)⁺; HPLC: tRet = 4.99 minutes (System1).

### Example 15: (4-Chloro-3-methoxy-phenyl)-[3-(3,4-dimethoxy-phenyl)-pyrazolo[1,5a] pyrimidin-5-yl]-amine:

The title compound is prepared as described in example 1, using 2-chloro-5-amino-anisole (TCl C1774) as amino starting material: Title compound: dark yellow crystals; MS(ESI⁺):m/z= 411.0 (M+H)⁺; HPLC: tRet = 7.08 minutes (System1).

### Example 16: trans-4-[{N-[3-(3,4-Dimethoxy-phenyl)-pyrazolo[1,5-a]pyrimidin-5-yl]-N-methyl-amino}-cyclohexan-1-yl]-methanol

The title compound is prepared as described in Example 1, using *trans-*(4-methylamino-cyclohexyl)-methanol as amino starting material

Title compound: bright yellow crystals; MS(ESI⁺):m/z= 397.1 (M+H)⁺; HPLC: tRet = 6.13 minutes (System1).

### Stage 16.1: trans- (4-Methylamino-cyclohexyl)-methanol

The title compound is prepared as described in example 7; Stage 7.1 , using tert.-butyl-*trans-*4-hydroxymethylcyclohexyl-carbamate (Albany Molecular Research Inc. A00060) instead. Title compound: white crystals; MS(ESI⁺):m/z= 144.0 (M+H)⁺.

### Example 17: N-(2,4-Difluoro-phenyl)-4-[3-(3,4-dimethoxy-phenyl)-pyrazolo[1,5-a]pyrimidin-5-ylamino]-benzamide:

The title compound is prepared as described in Example 1, using (4-aminophenyl)-N-(2,4-difluorophenyl)-formamide (ABCR, MOE1879) as amino starting material. Title compound: bright yellow crystals; MS(ESI⁺):m/z= 502 (M+H)⁺; HPLC: tRet = 6.81 minutes (System1).

### Example 18: Soft capsules

5000 soft gelatin capsules, each comprising as active ingredient 0.05 g of one of the compounds of formula I mentioned in the preceding Examples, are prepared as follows:

### Composition

Active ingredient 250 g

Lauroglycol 2 litres

Preparation process: The pulverized active ingredient is suspended in Lauroglykol® (propylene glycol laurate, Gattefossä S.A., Saint Priest, France) and ground in a wet pulverizer to produce a particle size of about 1 to 3 µm. 0.419 g portions of the mixture are then introduced into soft gelatin capsules using a capsule-filling machine.

## Claims

1. A compound of the formula I, 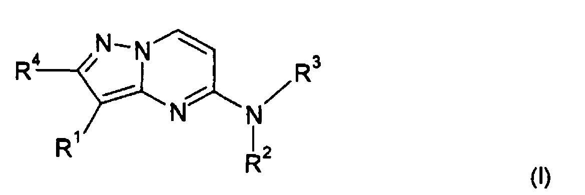 wherein
R¹ is di-C₁-C₇-alkoxy-phenyl;
R² is morpholino-C₁-C₇-alkyl, C₁-C₇-alkoxyphenyl, C₁-C₇-alkoxy-halo-phenyl, benzoylaminophenyl, [N'-mono- or N',N'-di-(C₁-C₇-alkyl)-aminocarbonyl]-phenyl , [difluorophenyl-aminocarbonyl]-phenyl, C₁-C₇-alkylcyclohexyl, hydroxyl-C₁-C₇-alkyl-cyclohexyl, hydroxyl-cyclohexyl, amino-cyclohexyl, benzyloxycarbonylamino-cyclohexyl, adamantan-1-yl or quinolyl;
R³ is hydrogen or methyl;
or R₂ and R₃ together with the binding nitrogen form piperidino which is unsubstituted or substituted by N-mono- or N,N-di-(C₁-C₇-alkyl)amino, and
R⁴ is hydrogen,
or a tautomer thereof or an N-oxide thereof, or a pharmaceutically acceptable salt, or a hydrate or solvate thereof.

2. A compound of the formula I according to claim 1,
wherein
R¹ is di-C₁-C₇-alkoxy-phenyl;
R² is 3-(morpholino)propyl, 4-C₁-C₇-alkoxyphenyl, 4-chloro-3-methoxyphenyl, 4-benzoylaminophenyl, 4-[N'-mono- or N',N'-di-(C₁-C₇-alkyl)-aminocarbonyl)-phenyl, 4-[2,4-difluorophenyl-aminocarbonyl]-phenyl, 2-, 3- or 4-C₁-C₇-alkylcyclohexyl, 2-, 3- or 4-hydroxyl-C₁-C₇-alkyl-cyclohexyl, 2-, 3- or 4-hydroxyl-cyclohexyl, 2-, 3- or 4-amino-cyclohexyl, 2-, 3- or 4-benzyloxycarbonylamino-cyclohexyl, adamantan-1-yl or quinolyl;
R³ is hydrogen or methyl;
or R₂ and R₃ together with the binding nitrogen form piperidino which is unsubstituted or substituted in the 4-position by N-mono- or N,N-di-(C₁-C₇-alkyl)amino, and
R⁴ is hydrogen,
or a tautomer thereof or an N-oxide thereof, or a pharmaceutically acceptable salt, or a hydrate or solvate thereof.

3. A compound of the formula I according to claim 1, selected from the group consisting of compounds with the following names:
*cis*-(1 S,2R)-2-[3-(3,4-dimethoxy-phenyl)-pyrazolo[1,5-a]pyrimidin-5-ylamino]-cyclohexanol;
*trans-*{4-[3-(3,4-dimethoxy-phenyl)-pyrazolo[1,5-a]pyrimidin-5-ylamino]-cyclohexyl}-carbamic acid benzyl ester;
*trans-*4-[3-(3,4-dimethoxy-phenyl)-pyrazolo[1,5-a]pyrimidin-5-ylamino]-cyclohexanol;
[3-(3,4-dimethoxy-phenyl)-pyrazolo[1,5-a]pyrimidin-5-yl]-(4-methoxy-phenyl)-amine;
*trans-*N-[3-(3.4-dimethoxy-phenyl)-pyrazolo[1,5-a]pyrimidin-5-yl]-cyclohexane-1,4-diamine;
adamantan-1-yl-[3-(3,4-dimethoxy-phenyl)-pyrazolo[1,5-a]pyrimidin-5-yl]-amine;
*trans-*4-{[3-(3,4-dimethoxy-phenyl)-pyrazolo[1,5-a]pyrimidin-5-yl]-methyl-amino}-cyclo-hexanol;
[3-(3,4-dimethoxy-phenyl)-pyrazolo[1,5-a]pyrimidin-5-yl]-(3-morpholin-4-yl-propyl)-amine;
{1-[3-(3,4-dimethoxy-phenyl)-pyrazolo[1,5-a]pyrimidin-5-yl]-piperidin-4-yl}-diethyl-amine;
*trans-*(1S,2R)-N-[3-(3,4-dimethoxy-phenyl)-pyrazolo[1,5-a]pyrimidin-5-yl]-cyclo-hexane-1,2-diamine;
4-[3-(3,4-dimethoxy-phenyl)-pyrazolo[1,5-a]pyrimidin-5-ylamino]-N-phenyl-benzamide;
N-(2-diethylamino-ethyl)-4-[3-(3,4-dimethoxy-phenyl)-pyrazolo[1,5-a]pyrimidin-5-ylamino]-benzamide;
*trans-*{4-[3-(3,4-dimethoxy-phenyl)-pyrazolo[1,5-a]pyrimidin-5-ylamino]-cyclohexan-1-yl}-methanol;
[3-(3,4-dimethoxy-phenyl)-pyrazolo[1,5-a]pyrimidin-5-yl]-quinolin-5-yl-amine;
(4-chloro-3-methoxy-phenyl)-[3-(3,4-dimethoxy-phenyl)-pyrazolo[1,5a] pyrimidin-5-yl]-amine;
*trans-*4-[{N-[3-(3,4-dimethoxy-phenyl)-pyrazolo[1,5-a]pyrimidin-5-yl]-N-methyl-amino}-cyclohexan-1-yl]-methanol; and
N-(2,4-difluoro-phenyl)-4-[3-(3,4-dimethoxy-phenyl)-pyrazolo[1,5-a]pyrimidin-5-ylamino]-benzamide;
or an N-oxide thereof, or a pharmaceutically acceptable salt, or a hydrate or solvate thereof.

4. A compound of the formula I, an N-oxide thereof, a tautomer thereof and/or a pharmaceutically acceptable salt thereof, according to any one of claims 1 to 3, for use in the treatment, including prophylactic treatment, of one or more diseases/disorders having an inflammatory component selected from conjunctivitis, keratoconjunctivitis sicca, and vernal conjunctivitis, allergic rhinitis, haemolytic anaemia, aplastic anaemia, pure red cell anaemia idiopathic thrombocytopenia, systemic lupus erythematosus, polychondritis, sclerodoma, Wegener granulamatosis, dermatomyositis, chronic active hepatitis, myasthenia gravis, Steven-Johnson syndrome, idiopathic sprue, autoimmune inflammatory bowel disease, endocrine opthalmopathy, Grave's disease, sarcoidosis, alveolitis, chronic hypersensitivity pneumonitis, multiple sclerosis, primary billiary cirrhosis, anterior- and posterior- uveitis, keratoconjunctivitis sicca and vernal keratoconjunctivitis, interstitial lung fibrosis, psoriatic arthritis and glomerulonephritis.

5. A combination comprising a compound of the formula I, according to any one of claims 1 to 3, an N-oxide thereof, a tautomer thereof and/or a pharmaceutically acceptable salt thereof, and one or more other pharmaceutically active compounds.

6. Use of a compound of the formula I, an N-oxide thereof, a tautomer thereof and/or a pharmaceutically acceptable salt thereof, according to any one of claims 1 to 3, for the manufacture of a medicament for the treatment, including prophylactic treatment, of one or more diseases or disorders having an inflammatory component selected from conjunctivitis, keratoconjunctivitis sicca, and vernal conjunctivitis, allergic rhinitis, haemolytic anaemia, aplastic anaemia, pure red cell anaemia idiopathic thrombocytopenia, systemic lupus erythematosus, polychondritis, sclerodoma, Wegener granulamatosis, dermatomyositis, chronic active hepatitis, myasthenia gravis, Steven-Johnson syndrome, idiopathic sprue, autoimmune inflammatory bowel disease, endocrine opthalmopathy, Grave's disease, sarcoidosis, alveolitis, chronic hypersensitivity pneumonitis, multiple sclerosis, primary billiary cirrhosis, anterior- and posterior-uveitis, keratoconjunctivitis sicca and vernal keratoconjunctivitis, interstitial lung fibrosis, psoriatic arthritis and glomerulonephritis.

7. A pharmaceutical preparation comprising a compound of the formula I, an N-oxide thereof, a tautomer thereof and/or a pharmaceutically acceptable salt thereof, according to any one of claims 1 to 3, and at least one pharmaceutically acceptable carrier.

8. A method or process for the manufacture of a pharmaceutical preparation, comprising mixing a compound of the formula I, an N-oxide thereof, a tautomer thereof and/or a pharmaceutically acceptable salt thereof, according to any one of claims 1 to 3, with at least one pharmaceutically acceptable carrier material.

9. A process for the manufacture of a compound according to any one of claims 1 to 3, comprising reacting a leaving group carrying compound of the formula II, 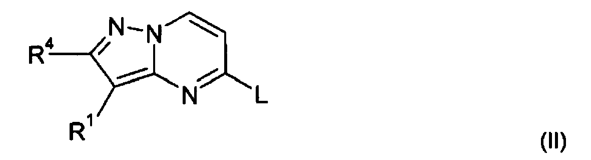 wherein R¹ and R⁴ are as defined for a compound of the formula I in any one of claims 1 to 3, and L is a leaving group, with an amino compound of the formula III, 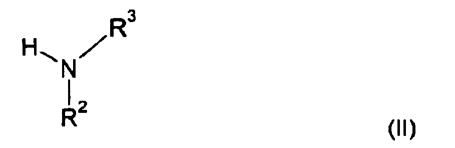 wherein R² and R³ are as defined for a compound of the formula I in any one of claims 1 to 3, where in the reaction functional groups in the starting materials can be present in protected form and in the obtainable compounds of the formula I carrying one or more protecting groups such protecting groups are removed;
and optionally, a compound of the formula I obtainable according to the reaction given above is converted into a different compound of the formula I, an obtainable salt of a compound of the formula I is converted into a different salt thereof, an obtainable free compound of the formula I is converted into a salt thereof, and/or an obtainable isomer of a compound of the formula I is separated from one or more different obtainable isomers of the formula I.

## Patentansprüche

1. Verbindung der Formel I 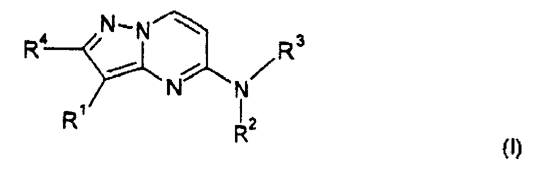 worin
R¹ für Di-C₁-C₇-alkoxyphenyl steht;
R² für Morphalino-C₁-C₇-alkyl, C₁-C₇-Alkoxyphenyl, C₁-C₇-Alkoxyhalogenphenyl, Benzoylaminophenyl, [N'-Mono- oder N',N'-Di(C₁-C₇-alkyl)aminocarbonyl]phenyl, [Difluorphenylaminocarbonyl]phenyl, C₁-C₇-Alkylcyclohexyl, Hydroxyl-C₁-C₇-alkylcyclohexyl, Hydroxylcyclohexyl, Aminocyclohexyl, Benzyloxycarbonylaminocyclohexyl, Adamantan-1-yl oder Chinolyl steht;
R³ für Wasserstoff oder Methyl steht; oder R² und R³ zusammen mit dem bindenden Stickstoff Piperidino bilden, das gegebenenfalls durch N-Mono- oder N, N-Di (C₁-C₇-alkyl) amino substituiert ist, und
R⁴ für Wasserstoff steht,
oder ein Tautomer davon oder ein N-Oxid davon oder ein pharmazeutisch unbedenkliches Salz oder ein Hydrat oder Solvat davon.

2. Verbindung der Formel I nach Anspruch 1, worin
R¹ für Di-C₁-C₇-alkoxyphenyl steht;
R² für 3-(Morpholino)propyl, 4-C₁-C₇-Alkoxyphenyl, 4-Chlor-3-methoxyphenyl, 4-Benzoylaminophenyl, 4-[N'-Mono- oder N',N'-Di(C₁-C₇-alkyl)aminocarbonyl]phenyl, 4-[2,4-Di-fluorphenylaminocarbonyl]phenyl, 2-, 3- oder 4-C₁-C₇-Alkylcyclohexyl, 2-, 3- oder 4-Hydroxyl-C₁-C₇-alkylcyclohexyl, 2-, 3- oder 4-Hydroxylcyclohexyl, 2-, 3- oder 4-Aminocyclohexyl, 2-, 3- oder 4-Benzyloxycarbonylaminocyclohexyl, Adamantan-1-yl oder Chinolyl steht;
R³ für Wasserstoff oder Methyl steht;
oder R² und R³ zusammen mit dem bindenden Stickstoff Piperidino bilden, das gegebenenfalls in der 4-Position durch N-Mono- oder N,N-Di(C₁-C₇-alkyl)amino substituiert ist, und
R⁴ für Wasserstoff steht,
oder ein Tautomer davon oder ein N-Oxid davon oder ein pharmazeutisch unbedenkliches Salz oder ein Hydrat oder Solvat davon.

3. Verbindung der Formel I nach Anspruch 1, ausgewählt aus der Gruppe bestehend aus Verbindungen mit den folgenden Namen:
*cis*-(1S,2R)-2-[3-(3,4-Dimethoxyphenyl)pyrazolo-[1,5-a]pyrimidin-5-ylamino]cyclohexanol;
*trans-*{4-[3-(3,4-Dimethoxyphenyl)pyrazolo[1,5-a]-pyrimidin-5-ylamino]cyclohexyl}carbamidsäure-benzylester;
*trans-*4-[3-(3,4-Dimethoxyphenyl)pyrazolo[1,5-a]-pyrimidin-5-ylamino]cyclohexanol;
[3-(3,4-Dimethoxyphenyl)pyrazolo[1,5-a]pyrimidin-5-yl]-4-(methoxyphenyl)amin;
*trans-*N-[3-(3,9-Dimethoxyphenyl)pyrazolo[1,5-a]-pyrimidin-5-yl]cyclohexan-1,4-diamin;
Adamantan-1-yl[3-(3,4-dimethoxyphenyl)pyrazolo-[1,5-a]pyrimidin-5-yl]amin
*trans-*4-{[3-(3,4-Dimethoxyphenyl)pyrazolo[1,5-a]-pyrimidin-5-yl]methylamino}cyclohexanol;
[3-(3,4-Dimethoxyphenyl)pyrazolo[1,5-a]pyrimidin-5-yl](3-morpholin-4-ylpropyl)amin;
{1-[3-(3,4-Dimethoxyphenyl)pyrazolo[1,5-a]-pyrimidin-5-yl]piperidin-4-yl}diethylamin;
*trans-*(1S,2R)-N-[3-(3,4-Dimethoxyphenyl)pyrazolo-[1,5-a]pyrimidin-5-yl]cyclohexan-1,2-diamin;
4-[3-(3,4-Dimethoxyphenyl)pyrazolo[1,5-a]-pyrimidin-5-ylamino]-N-phenylbenzamid;
N-(2-Diethylaminoethyl)-4-[3-(3,4-dimethoxyphenyl)pyrazolo[1,5-a]pyrimidin-5-ylamino]benzamid;
*trans-*{4-[3-(3,4-Dimethoxyphenyl)pyrazolo[1,5-a]-pyrimidin-5-ylamino]cyclohexan-1-yl}methanol;
[3-(3,4-Dimethoxyphenyl)pyrazolo[1,5-a]pyrimidin-5-yl]chinolin-5-ylamin;
(4-Chlor-3-methoxyphenyl)-[3-(3,4-dimethoxyphenyl)pyrazolo[1,5-a]pyrimidin-5-yl]amin;
*trans-*4-[{N-[3-(3,4-Dimethoxyphenyl)pyrazolo[1,5-a]pyrimidin-5-yl]-N-methylamino}cyclohexan-1-yl]-methanol und
N-(2,4-Difluorphenyl)-4-[3-(3,4-dimethoxyphenyl)-pyrazolo[1,5-a]pyrimidin-5-ylamino]benzamid;
oder ein N-Oxid davon oder ein pharmazeutisch unbedenkliches Salz oder ein Hydrat oder Solvat davon.

4. Verbindung der Formel I, ein N-Oxid davon, ein Tautomer davon und/oder ein pharmazeutisch unbedenkliches Salz davon nach einem der Ansprüche 1 bis 3 zur Verwendung bei der Behandlung, einschließlich der prophylaktischen Behandlung, einer oder mehrerer Erkrankungen/Störungen mit einer entzündlichen Komponente, ausgewählt unter Konjunktivitis, Keratoconjunctivitis sicca und Frühjahrskonjunktivitis, allergischer Rhinitis, hämolytischer Anämie, aplastischer Anämie, chronischer kongenitaler aregenerativer Anämie und idiopathischer Thrombocytopenie, systemischem Lupus erythematodes, Polychondritis, Skleroderm, Wegener-Granulamatose, Dermatomyositis, chronischer aktiver Hepatitis, Myasthenia gravis, Steven-Johnson-Syndrom, idiopathischer Sprue, autoimmuner entzündlicher Darmerkrankung, endokriner Ophthalmopathie, Basedow-Krankheit, Sarkoidose, Alveolitis, chronischer hypersensibler Pneumonitis, multipler Sklerose, primärer biliärer Zirrhose, Uveitis anterior und posterior, Keratoconjunctivitis sicca und Frühjahrskeratokonjunktivitis, interstitieller Lungenfibrose, Arthritis psoriatica und Glomerulonephritis.

5. Kombination, umfassend eine Verbindung der Formel I nach einem der Ansprüche 1 bis 3, ein N-Oxid davon, ein Tautomer davon und/oder ein pharmazeutisch unbedenkliches Salz davon und eine oder mehrere andere pharmazeutisch wirksame Verbindungen.

6. Verwendung einer Verbindung der Formel I, eines N-Oxids davon, eines Tautomers davon und/oder eines pharmazeutisch unbedenklichen Salzes davon nach einem der Ansprüche 1 bis 3 zur Herstellung eines Arzneimittels zur Behandlung, einschließlich der prophylaktischen Behandlung, einer oder mehrerer Erkrankungen/Störungen mit einer entzündlichen Komponente, ausgewählt unter Konjunktivitis, Keratoconjunctivitis sicca und Frühjahrskonjunktivitis, allergischer Rhinitis, hämolytischer Anämie, aplastischer Anämie, chronischer kongenitaler aregenerativer Anämie und idiopathischer Thrombocytopenie, systemischem Lupus erythematodes, Polychondritis, Skleroderm, Wegener-Granulamatose, Dermatomyositis, chronischer aktiver Hepatitis, Myasthenia gravis, Steven-Johnson-Syndrom, idiopathischer Sprue, autoimmuner entzündlicher Darmerkrankung, endokriner Ophthalmopathie, Basedow-Krankheit, Sarkoidose, Alveolitis, chronischer hypersensibler Pneumonitis, multipler Sklerose, primärer biliärer Zirrhose, Uveitis anterior und posterior, Keratoconjunctivitis sicca und Frühjahrskeratokonjunktivitis, interstitieller Lungenfibrose, Arthritis psoriatica und Glomerulonephritis.

7. Pharmazeutische Zubereitung, umfassend eine Verbindung der Formel I, ein N-Oxid davon, ein Tautomer davon und/oder ein pharmazeutisch unbedenkliches Salz davon nach einem der Ansprüche 1 bis 3 und mindestens einen pharmazeutisch unbedenklichen Träger.

8. Methode oder Verfahren zur Herstellung einer pharmazeutischen Zubereitung, bei der bzw. dem man eine Verbindung der Formel I, ein N-Oxid davon, ein Tautomer davon und/oder ein pharmazeutisch unbedenkliches Salz davon nach einem der Ansprüche 1 bis 3 mit mindestens einem pharmazeutisch unbedenklichen Träger mischt.

9. Verfahren zur Herstellung einer Verbindung der Formel I nach einem der Ansprüche 1 bis 3, bei dem man eine eine Abgangsgruppe tragende Verbindung der Formel II 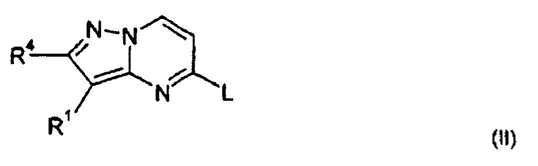 worin R¹ und R⁴ die für eine Verbindung der Formel I in einem der Ansprüche 1 bis 3 angegebene Bedeutung besitzen und L für eine Abgangsgruppe steht, mit einer Aminoverbindung der Formel III 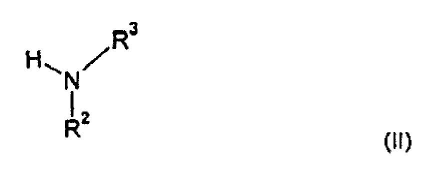 worin R² und R³ die für eine Verbindung der Formel I in einem der Ansprüche 1 bis 3 angegebene Bedeutung besitzen,
umsetzt, wobei funktionelle Gruppen in den Ausgangsstoffen bei der Umsetzung in geschützter Form vorliegen können und in den erhältlichen Verbindungen der Formel I, die eine oder mehrere Schutzgruppen tragen, derartige Schutzgruppen abgespalten werden;
und gegebenenfalls eine gemäß der oben angegebenen Umsetzung erhältliche Verbindung der Formel I in eine andere Verbindung der Formel I umwandelt, ein erhältliches Salz einer Verbindung der Formel I in ein anderes Salz davon umwandelt, eine erhältliche freie Verbindung der Formel I in ein Salz davon umwandelt und/oder ein erhältliches Isomer einer Verbindung der Formel I von einem oder mehreren anderen erhältlichen Isomeren der Formel I trennt.

## Revendications

1. Composé de formule I, 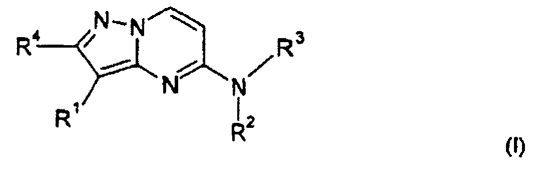 où
R¹ représente un groupement di-(alkoxy en C₁-C₇)-phényle ;
R² représente un groupement morpholino-(alkyle en C₁-C₇), (alkoxy en C₁-C₇)-phényle, (alkoxy en C₁-C₇)-halogénophényle, benzoylaminophényle, [N'-mono- ou N',N'-di- (alkyle en C₁-C₇)-aminocarbonyl] -phényle, [difluorophényl-aminocarbonyl]--phényle, (alkyle en C₁-C₇)-cyclohexyle, hydroxyl-(alkyle en C₁-C₇)-cyclohexyle, hydroxylcyclohexyle, amino-cyclohexyle, benzyloxycarbonylamino-cyclohexyle, adamantan-1-yle ou quinoléyle ;
R³ représente un atome d'hydrogène ou un groupement méthyle ;
ou R₂ et R₃ forment ensemble et avec l'atome d'azote auquel ils sont liés un groupement pipéridino éventuellement substitué par des groupements N-mono- ou N,N-di-(alkyle en C₁-C₇)amino, et
R⁴ représente un atome d'hydrogène,
ou l'un de ses tautomères ou N-oxydes, ou leurs sels, hydrates ou solvates de qualité pharmaceutique.

2. Composé de formule I conforme à la revendication 1, où
R¹ représente un groupement di-(alkoxy en C₁-C₇)-phényle ;
R² représente un groupement 3-(morpholino)propyle, 4-(alkoxy en C₁-C₇)-phényle, 4-chloro-3-méthoxyphényle, 4-benzoylamino-phényle, 4-[N'-mono- ou N',N'-di-(alkyle en C₁-C₇)-aminocarbonyl)-phényle, 4-[2,4-difluorophényl-aminocarbonyl]-phényle, 2-, 3- ou 4-(alkyle en C₁-C₇)-cyclohexyle, 2-, 3- ou 4-hydroxyl-(alkyle en C₁-C₇)-cyclohexyle, 2-, 3- ou 4-hydroxylcyclohexyle, 2-, 3- ou 4-amino-cyclohexyle, 2-, 3- ou 4-benzyloxycarbonylamino-cyclohexyle, adamantan-1-yle ou quinoléyle ;
R³ représente un atome d'hydrogène ou un groupement méthyle ;
ou R₂ et R₃ forment ensemble et avec l'atome d'azote auquel ils sont liés un groupement pipéridino éventuellement substitué en position 4 par des groupements N-mono- ou N,N-di-(alkyle en C₁-C₇)amino, et R⁴ représente un atome d'hydrogène,
ou l'un de ses tautomères ou N-oxydes, ou leurs sels, hydrates ou solvates de qualité pharmaceutique.

3. Composé de formule I conforme à la revendication 1, choisi dans le groupe constitué par les composés suivants :
*cis*-(1S,2R)-2-[3-(3,4-diméthoxy-phényl)-pyrazolo[1,5-a]pyrimidin-5-ylamino]-cyclohexanol ;
*trans-*{4-[3-(3,4-diméthoxy-phényl)-pyrazolo[1,5-a]pyrimidin-5-ylamino]-cyclohexyl}-carbamate de benzyle ;
*trans-*4-[3-(3,4-diméthoxy-phényl)-pyrazolo[1,5-a]pyrimidin-5-ylamino]-cyclohexanol ;
[3-(3,4-diméthoxy-phényl)-pyrazolo[1,5-a]pyrimidin-5-yl]-(4-méthoxy-phényl)-amine ;
*trans-*N-[3-(3,4-diméthoxy-phényl)-pyrazolo[1,5-a]pyrimidin-5-yl]-cyclohexane-1,4-diamine ;
adamantan-1-yl-[3-(3,4-diméthoxy-phényl)-pyrazolo[1,5-a]pyrimidin-5-yl]-amine ;
*trans-*4-{[3-(3,4-diméthoxy-phényl)-pyrazolo[1,5-a]pyrimidin-5-yl]-méthyl-amino}-cyclohexanol ;
[3-(3,4-diméthoxy-phényl)-pyrazolo[1,5-a]pyrimidin-5-yl]-(3-morpholin-4-yl-propyl)-amine ;
(1-[3-(3,4-diméthoxy-phényl)-pyrazolo[1,5-a]pyrimidin-5-yl]-pipéridin-4-yl)-diéthyl-amine ;
*trans-*(1S,2R)-N-[3-(3,4-diméthoxy-phényly-pyrazolo[1,5-a]pyrimidin-5-yl]-cyclo-hexane-1,2-diamine ;
4-[3,4-diméthoxy-phényl)-pyrazolo[1,5-a]pyrimidin-5-ylamino]-N-phényl-benzamide ;
N-(2-diéthylamino-éthyl)-4-[3-(3,4-diméthoxy-phényl)-pyrazolo[1,5-a]pyrimidin-5-ylamino]-benzamide ;
*trans-*{4-[3-(3,4-diméthoxy-phényl)-pyrazolo[1,5-a]pyrimidin-5-ylamino]-cyclohexan-1-yl}-méthanol ;
[3-(3,4-diméthoxy-phényl)-pyrazolo[1,5-a]pyrimidin-5-yl]-quinoléin-5-yl-amine ;
(4-chloro-3-méthoxy-phényl)-[3-(3,4-diméthoxy-phényl)-pyrazolo[1,5a]pyrimidin-5-yl]-amine ;
*trans-*4-[{N-[3-(3,4-diméthoxy-phényl)-pyrazolo[1,5-a]pyrimidin-5-yl]-N-méthyl-amino)-cyclohexan-1-yl]-méthanol ; et
N-(2,4-difluoro-phényl)-4-[3-(3,4-diméthoxy-phényl)-pyrazolo[1,5-a]pyrimidin-5-ylamïno]-benzamide ;
ou leurs N-oxydes, ou leurs sels, hydrates ou solvates sels de qualité pharmaceutique.

4. Composé de formule I, l'un de ses N-oxydes, l'un de ses tautomères et/ou l'un de ses sels de qualité pharmaceutique, conforme à l'une quelconque des revendications 1 à 3, pour emploi dans le traitement, y compris le traitement prophylactique, d'un ou de plusieurs troubles ou pathologies à composante inflammatoire choisis parmi les suivantes :
conjonctivite, kérato-conjonctivite sèche et kérato-conjonctivite vernale, rhinite allergique, anémie hémolytique, anémie aplasique, érythroblastopénie et
thrombocytopénie idiopathique, lupus érythémateux disséminé, polychondrite, sclérodermie, granulomatose de Wegener, dermatomyosite, hépatite chronique active,
myasthénie grave, syndrome de Steven-Johnson, sprue idiopathique, affections abdominales inflammatoires auto-immunes, ophtalmopathie endocrinienne, maladie de Basedow, sarcoïdose, alvéolite, pneumopathie chronique d'hypersensibilité, sclérose en plaques, cirrhose biliaire primitive, uvéite antérieure et postérieure,
kérato-conjonctivite sèche et kérato-conjonctivite vernale, fibrose pulmonaire interstitielle, arthrite psoriasique et glomérulonéphrite.

5. Combinaison comprenant un composé de formule I conforme à l'une quelconque des revendications 1 à 3, l'un de ses N-oxydes, l'un de ses tautomères et/ou l'un de ses sels de qualité pharmaceutique, avec un ou plusieurs autres principes actifs pharmaceutiques.

6. Emploi d'un composé de formule I, l'un de ses N-oxydes, l'un de ses tautomères et/ou l'un de ses sels de qualité pharmaceutique, conforme à l'une quelconque des revendications 1 à 3, dans la fabrication d'un médicament destiné au traitement, y compris au traitement prophylactique, d'un ou de plusieurs troubles ou pathologies à composante inflammatoire choisis parmi les suivants : conjonctivite, kérato-conjonctivite sèche et kérato-conjonctivite vernale, rhinite allergique, anémie hémolytique, anémie aplasique, érythroblastopénie et thrombocytopénie idiopathique, lupus érythémateux disséminé, polychondrite, sclérodermie, granulomatose de Wegener, dermatomyosite, hépatite chronique active, myasthénie grave, syndrome de Steven-Johnson, sprue idiopathique, affections abdominales inflammatoires auto-immunes, ophtalmopathie endocrinienne, maladie de Basedow, sarcoïdose, alvéolite, pneumopathie chronique d'hypersensibilité, sclérose en plaques, cirrhose biliaire primitive, uvéite antérieure et postérieure, kérato-conjonctivite sèche et kérato-conjonctivite vernale, fibrose pulmonaire interstitielle, arthrite psoriasique et glomérulonéphrite.

7. Préparation pharmaceutique comprenant un composé de formule I, l'un de ses N-oxydes, l'un de ses tautomères et/ou l'un de ses sels de qualité pharmaceutique conforme à l'une quelconque des revendications 1 à 3 avec au moins un vecteur de qualité pharmaceutique.

8. Méthode ou procédé de fabrication d'une préparation pharmaceutique comprenant le mélangeage d'un composé de formule I, de l'un de ses N-oxydes, de l'un de ses tautomères et/ou de l'un de ses sels de qualité pharmaceutique conforme à l'une quelconque des revendications 1 à 3 avec au moins un vecteur de qualité pharmaceutique.

9. Procédé de fabrication d'un composé conforme à l'une quelconque des revendications 1 à 3, ledit procédé comprenant la réaction d'un composé de formule II portant un groupement partant, 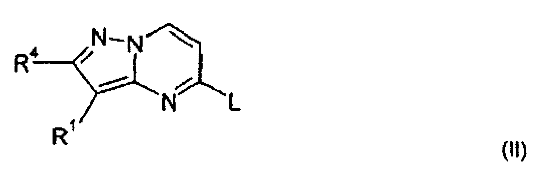 où R¹ et R⁴ sont tels que définis pour un composé de formule I dans l'une quelconque des revendications 1 à 3, et L représente un groupement partant, avec un composé amino de formule III, 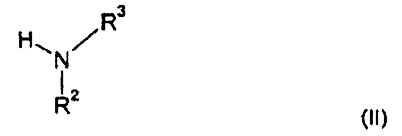 où R² et R³ sont tels que définis pour un composé de formule I dans l'une quelconque des revendications 1 à 3,
les groupements fonctionnels des produits de départ de la réaction pouvant être présents sous forme protégée et, dans les composés de formule I pouvant être obtenus et portant un ou plusieurs groupements protecteurs, de tels groupements protecteurs étant clivés ;
et, éventuellement, un composé de formule I pouvant être obtenu selon la réaction ci-avant est converti en un composé différent de formule I, un sel de composé de formule I pouvant être obtenu est converti en un sel différent dudit composé, un composé libre de formule I pouvant être obtenu est converti en l'un de ses sels, et/ou un isomère d'un composé de formule I pouvant être obtenu est séparé d'un de plusieurs isomères différents de formule I pouvant être obtenus.
